# FASCICULE DE BREVET EUROPEEN

(11) **EP 3 516 070 B1**
(45) Date de publication et mention de la délivrance du brevet: **15.12.2021**
(21) Numéro de dépôt: 17768146.7
(22) Date de dépôt: 19.09.2017
(51) Int. Cl.: C12Q 1/68, C12Q 1/6883

(54) **METHODE D'EVALUATION DU POTENTIEL D'IRRITATION OCULAIRE DE PRODUITS CHIMIQUES**
VERFAHREN ZUR EVALUIERUNG DER OKULAREN EMPFINDLICHKEIT VON CHEMISCHEN PRODUKTEN
METHOD FOR EVALUATING THE OCULAR IRRITABILITY POTENTIAL OF CHEMICAL PRODUCTS

(30) Priorité: 19.09.2016 FR 1658746
(43) Date de publication de la demande: 31.07.2019
(73) Titulaire: Immunosearch, 06130 Le Plan De Grasse (FR)
(72) Inventeur: GROUX, Hervé, 06650 Le Rouret (FR); COTTREZ, Françoise, 06650 Le Rouret (FR); ALEPEE, Nathalie, 93190 Livry Gargan (FR); LEBLANC, Virginie, 94230 Cachan (FR)
(74) Mandataire: Regimbeau
(86) Numéro de dépôt international: PCT/EP2017/073675
(87) Numéro de publication internationale: WO 2018/050927

(56) Documents cités:
- CA-A1- 2 318 641
- FR-A1- 2 957 090
- YAMAMOTO NAOKI ET AL: "Establishment of a new immortalized human corneal epithelial cell line (iHCE-NY1) for use in evaluating eye irritancy by in vitro test methods", IN VITRO CELLULAR & DEVELOPMENTAL BIOLOGY. ANIMAL, SPRINGER US, NEW YORK, vol. 52, no. 7, 29 avril 2016 (2016-04-29) , pages 742-748, XP036030366, ISSN: 1071-2690, DOI: 10.1007/S11626-016-0038-9 [extrait le 2016-04-29]
- RÖNKKÖ SEPPO ET AL: "Human corneal cell culture models for drug toxicity studies", DRUG DELIVERY AND TRANSLATIONAL RESEARCH, SPRINGER, GERMANY, vol. 6, no. 6, 9 septembre 2016 (2016-09-09), pages 660-675, XP036092774, ISSN: 2190-393X, DOI: 10.1007/S13346-016-0330-Y [extrait le 2016-09-09]
- KHOH-REITER SU ET AL: "Evaluation of the cytotoxic effects of ophthalmic solutions containing benzalkonium chloride on corneal epithelium using an organotypic 3-D model", BMC OPHTHALMOLOGY, BIOMED CENTRAL, LONDON, GB, vol. 9, no. 1, 28 juillet 2009 (2009-07-28), page 5, XP021057710, ISSN: 1471-2415, DOI: 10.1186/1471-2415-9-5
- MELONI M ET AL: "Occludin gene expression as an early in vitro sign for mild eye irritation assessment", TOXICOLOGY IN VITRO, ELSEVIER SCIENCE, GB, vol. 24, no. 1, 1 février 2010 (2010-02-01), pages 276-285, XP026812650, ISSN: 0887-2333 [extrait le 2009-09-01]

## Description

La présente invention concerne une méthode d'évaluation et de catégorisation du potentiel d'irritation oculaire de produits chimiques.

Pour évaluer l'innocuité de produits chimiques au niveau de l'œil, on connait le test de Draize qui est un test toxicologique invasif basé sur un protocole d'expérimentation animale, mis au point en 1944 par John H. Draize et Jacob M. Spines, toxicologues de la Food and Drug Administration (FDA). Le test consiste à tester un produit dans l'oeil d'un animal afin d'évaluer son innocuité oculaire.

En pratique, il consiste à appliquer 0,1 millilitre ou 0,1 gramme d'une substance dans l'oeil d'un animal conscient, souvent un lapin, pendant une à vingt-quatre heures. On observe ensuite, pendant une durée qui peut aller jusqu'à 21 jours, l'éventuelle apparition d'une opacité de la cornée, d'une conjonctivite, d'un oedème, ou encore de sécrétions.

L'utilisation d'animaux en laboratoire dans le cadre du test de Draize est un sujet combattu par les défenseurs des animaux. Au sein de l'Union européenne, l'expérimentation animale est encadrée par la directive 2010/63, qui encourage le développement de méthodes de tests alternatives.

Les produits chimiques sont classifiés selon un système globalisé et mis en place par les Nations Unies connu sous le terme United Nations Globally Harmonized Sytem et concernant les propriétés d'irritation oculaire, les produits sont classés en plusieurs catégories. La catégorie 1 concerne les produits causant des dommages dont on ne constate pas la réversibilité après 21 jours, la catégorie 2A concerne les produits irritants pour les yeux de manière réversible avant 21 jours et la catégorie 2B concerne les produits moyennement irritants pour les yeux de manière réversible avant 21 jours. Les produits non irritants ne sont pas classifiés. La législation Européenne combine les catégories 2A et 2B en une seule catégorie 2 qualifiée d'irritante de manière réversible pour les yeux.

On connaît aussi un procédé pour estimer l'inconfort oculaire ou le potentiel irritant d'un produit chimique tel qu'un produit cosmétique. Ce procédé comprend la culture in vitro d'un modèle d'épiderme, le dépôt du produit que l'on souhaite étudier sur modèle d'épiderme cultivé, et la quantification du nombre de cellules vivantes survivantes afin d'évaluer l'inconfort oculaire ou le potentiel irritant de celui-ci. Un tel procédé, développé en remplacement de l'expérimentation animale, présente l'inconvénient d'être long et coûteux et peu discriminant. De plus cette méthode ne constitue pas une méthode de remplacement intégrale, puisqu'elle ne permet pas de détecter de façon fiable toute la gamme d'irritations intermédiaires.

Ainsi, il apparait qu'il existe un besoin en un test ou méthode permettant d'une part de remplacer le test de Draize et de fournir en particulier une méthode permettant de différencier et de catégoriser rapidement les composés causant une irritation oculaire irréversible à 21 jours de ceux dont les effets sont réversibles avant 21 jours.

Les inventeurs de la présente demande se sont donc penchés sur le développement d'un test in vitro pour la détection du potentiel irritant de produits chimiques combinant un modèle cellulaire cornéal à une sélection de marqueurs moléculaires prédictifs et qualitatifs permettant de classifier les composés en 3 catégories, à savoir dommages non réversibles aux yeux 21 jours après application (catégorie 1), dommages réversibles aux yeux 21 jours après application (catégorie 2) et non irritation (no Catégorie).

De manière surprenante, les inventeurs ont mis en évidence que la réponse consécutive à l'action d'une substance irritante se fait directement sur un épithélium cornéal reconstruit in vitro, et que le degré d'irritation, et la qualification de cette irritation d'une molécule, peut être déterminé grâce à l'utilisation de biomarqueurs spécifiques de l'irritation oculaire. Il est notable de remarquer, et cela constitue une caractéristique surprenante de la présente méthode, que les marqueurs moléculaires mise en œuvre n'ont jamais été décrits ou divulgués en relation avec l'irritation oculaire.

Les Inventeurs ont mis en évidence qu'un epithélium cornéal reconstruit in vitro constitue un modèle suffisant pour mettre en évidence des biomarqueurs spécifiques de l'irritation oculaire chez l'homme, et que ces biomarqueurs permettent en outre de prédire le degré d'irritation et donc de classifier de manière prédictive les produits testés selon les 3 catégories telles qu'indiquées plus avant.

Ainsi, la présente invention se rapporte à une méthode d'évaluation du potentiel d'irritation oculaire d'un composé test, comprenant les étapes de:
a) mise en contact d'un composé test avec un échantillon cornéal reconstruit in vitro;
b) mesure de l'expression d'au moins un gène choisi dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61, caractérisé en ce que :
   - la mise en contact du composé test à l'étape a) est réalisée avec le composé test sous forme solide, dans laquelle l'étape b) comprend la mesure de l'expression d'au moins un gène choisi dans le groupe constitué par : IL-24, IL-23R, DDIT3, MMP8, DLK1, HAS1, CYR61, IL-1R2, CLEC4D, ICAM2, CASP1, MUC13 et MUC4.
   Ou
   - la mise en contact du composé test à l'étape a) est réalisée avec le composé test sous forme liquide, dans laquelle l'étape b) comprend la mesure de l'expression d'au moins un gène choisi dans le groupe constitué par : HSP90AA1, CASP1, DLK1, CLEC4D, IL-24, SLIT2, HSPA1A, FSHR, IL-1R2 et CCND1,
   - et en ce qu'elle comprend en outre une étape c) de détermination d'un index d'irritation oculaire d'un composé test.

Préférentiellement, la méthode comprend aussi une étape d) de catégorisation dudit composé comme présentant un potentiel d'irritation oculaire selon la valeur de l'index d'irritation oculaire obtenu.

De préférence, la méthode selon la présente invention est une méthode *in vitro.*

Tel qu'utilisé ici, le terme « échantillon cornéal reconstruit in vitro » se réfère à un échantillon de cellules épithéliales de cornée cultivées en milieu de culture défini ou tout modèle utilisant des cellules épithéliales squameuses humaines et présentant une morphologie similaire à la cornée humaine, tels que les modèles de cornée in vitro du type de ceux commercialisés sous la marque EpiOcular^{®}.

Plus particulièrement encore, cet « échantillon cornéal reconstruit in vitro » est un échantillon comprenant, ou constitué par, des cellules épithéliales de cornée immortalisées, cultivées en milieu de culture défini et disposées en couche fine sur une membrane synthétique à l'interface eau-air.

De préférence, lesdites cellules épithéliales de cornée immortalisées sont des cellules humaines.

Dans un mode de réalisation particulier, cet « échantillon cornéal reconstruit in vitro » est un échantillon HCE SkinEthic^{®} commercialisé par la société Episkin (Lyon, France) qui est un épithélium de cornée reconstruit constitué de kératinocytes de cornée humaine, particulièrement des cellules transformées pour être rendues immortelles.

Au sens de la présente invention, la mesure de l'expression dudit au moins un gène de l'étape b) permet de déterminer le niveau d'expression dudit gène.

Le composé test peut être un composé de nature, structure et origine variées, notamment un composé biologique, un composé chimique, synthétique, etc.

Le composé test peut être tout produit qui se présente sous une forme isolée ou en mélange avec d'autres produits. Le composé test peut être défini en termes de structure et/ou de composition ou être défini sur le plan fonctionnel. Le composé test peut, par exemple, être un produit isolé et structurellement défini, un produit isolé de structure indéfinie, un mélange de produits connus et caractérisés ou une composition comprenant un ou plusieurs produits. Un ou plusieurs composés peuvent ainsi être testés, en mélange ou de manière séparée.

La présente invention est particulièrement adaptée à l'identification d'un nombre important de composés. Ce criblage simple et efficace peut être accompli en un laps de temps très court. Les méthodes décrites peuvent en particulier être partiellement automatisées, autorisant ainsi le criblage efficace et simultané de composés divers et nombreux, soit sous forme de mélange soit sous forme séparée.

De préférence, dans la méthode selon la présente invention, le niveau d'expression dudit gène est évalué par mesure du niveau d'expression du polypeptide codé par ledit gène ou un fragment de celui-ci, ou par mesure du niveau d'expression de l'ARNm dudit gène ou d'un fragment de celui-ci.

Dans un mode de réalisation préféré, l'expression dudit au moins un gène est effectuée par analyse de l'expression de transcrits d'ARNm ou de précurseurs d'ARNm, tel qu'un ARN natif, dudit gène. Ladite analyse peut être réalisée en préparant l'ARNm/ADNc de cellules d'un échantillon biologique d'un patient, et hybridation de l'ARNm /ADNc avec un polynucléotide de référence. L'ARNm/ADNc préparé peut être utilisé dans une analyse par hybridation ou amplification qui inclut, sans s'y limiter, les analyses Southern et Northen, les analyses par PCR (« polymerase chain reaction »), telle que la PCR quantitative (Taqman) et l'utilisation de sondes (« probes arrays ») telles que les matrices ADN GeneChip^{®}(AFFYMETRIX).

Avantageusement, l'analyse de l'expression du niveau d'ARNm transcrit dudit au moins un gène implique un procédé d'amplification des acides nucléiques, comme par exemple la RT-PCR (mode de réalisation expérimental décrit dans le Brevet US 4,683,202), la réaction en chaîne par la ligase (BARANY, Proc. Natl. Acad. Sci. USA, vol.88, p: 189-193, 1991), la réplication de séquences auto-entretenue (« self sustained sequence replication ») (GUATELLI et al., Proc. Natl. Acad. Sci. USA, vol.87, p: 1874-1878, 1990), le système d'amplification transcriptionnelle. (KWOH et al., Proc. Natl. Acad. Sci. USA, vol.86, p: 1173-1177, 1989), la « Q-Beta Replicase » (LIZARDI et al., Biol. Technology, vol.6, p: 1197, 1988), la « rolling circle replication » (U. S. Patent No. 5,854,033) ou toute autre méthode d'amplification d'acides nucléiques, suivie d'une étape de détection des molécules amplifiées par des techniques bien connues de l'homme du métier. Ces modes de détection sont particulièrement utiles pour la détection de molécules d'acides nucléiques en très faibles quantités.

Ainsi, selon un mode de réalisation préféré, la méthode selon la présente invention comprend une étape supplémentaire d'amplification de l'ARNm ou de l'ADNc dudit gène, de la séquence complémentaire de celle-ci ou d'un fragment de celle-ci.

Telles qu'utilisées ici, les amorces d'amplifications sont définies comme étant une paire de molécules d'acides nucléiques qui peuvent s'apparier respectivement aux régions 3' et 5' d'un gène de façon spécifique (brins positif et négatif, ou inversement) et encadrent une courte région dudit gène. De manière générale, les amorces d'amplification ont une longueur de 10 à 30 nucléotides et permettent l'amplification d'une région d'une longueur comprise entre 50 et 200 nucléotides.

Dans un autre mode de réalisation préféré, la mesure de l'expression dudit au moins un gène est réalisée par mesure du niveau d'expression du polypeptide codé par ledit gène. Ladite analyse peut être réalisée en utilisant un anticorps (par exemple, un anticorps radiomarqué, marqué avec un chromophore, un fluorophore, ou une enzyme), un dérivé d'anticorps (par exemple un anticorps conjugué à un substrat ou à une protéine ou un ligand d'une protéine d'un couple ligand/protéine (par exemple biotine-streptavidine)) ou un fragment d'anticorps (par exemple un anticorps à une seule chaîne, un domaine hypervariable d'un anticorps isolé, etc.) qui se lie spécifiquement au polypeptide codé par ledit gène. Lesdites analyses peuvent être réalisées par de nombreuses techniques à la portée de l'homme du métier incluant, sans s'y limiter, les tests immunologiques basés sur l'utilisation d'activité enzymatique (« enzyme immunoassay » EIA), les tests immunologiques basés sur l'utilisation d'isotopes radioactifs (RIA), l'analyse par Western blot et les tests ELISA (« enzyme linked immunoabsorbant assay »).

Au sens de la présente invention, on entend par « polypeptide » une séquence comprenant au moins deux acides aminés, et les termes « polypeptide », « peptide » et « protéine » peuvent être indifféremment utilisés.

Par « fragment de l'ARNm ou de l'ADNc », on entend une séquence d'au moins 50 acides nucléiques, à titre d'exemple d'au moins 100 ou 150 acides nucléiques, de préférence d'au moins 200 acides nucléiques, à titre d'exemple d'au moins 250 ou 350 acides nucléiques, et de manière particulièrement préférée un polypeptide d'au moins 400 acides nucléiques.

Par « fragment du polypeptide », on entend une séquence d'au moins 50 acides aminés, à titre d'exemple d'au moins 100 ou 150 acides aminés, de préférence d'au moins 200 acides aminés, à titre d'exemple d'au moins 250 ou 350 acides aminés, et de manière particulièrement préférée un polypeptide d'au moins 400 acides aminés.

Préférentiellement, ladite méthode permet d'évaluer le potentiel d'irritation oculaire d'un composé test, dans laquelle l'étape b) comprend la mesure de l'expression d'au moins deux gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins trois gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins quatre gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins cinq gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins six gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins sept gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins huit gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins neuf gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins dix gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins onze gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins douze gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

Préférentiellement, ladite méthode permet d'évaluer le potentiel d'irritation oculaire d'un composé test, dans laquelle l'étape b) comprend la mesure de l'expression d'au moins treize gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.

La méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression d'au moins un gène choisi dans le groupe constitué par : HSP90AA1, CASP1, DLK1, CLEC4D, IL-24, SLIT2, HSPA1A, FSHR, IL-1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression d'au moins deux gènes choisis dans le groupe constitué par : HSP90AA1, CASP1, DLK1, CLEC4D, IL-24, SLIT2, HSPA1A, FSHR, IL-1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression d'au moins trois gènes choisis dans le groupe constitué par : HSP90AA1, CASP1, DLK1, CLEC4D, IL-24, SLIT2, HSPA1A, FSHR, IL-1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression d'au moins quatre gènes choisis dans le groupe constitué par : HSP90AA1, CASP1, DLK1, CLEC4D, IL-24, SLIT2, HSPA1A, FSHR, IL-1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression d'au moins cinq gènes choisis dans le groupe constitué par : HSP90AA1, CASP1, DLK1, CLEC4D, IL24, SLIT2, HSPA1A, FSHR, IL1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression d'au moins six gènes choisis dans le groupe constitué par : HSP90AA1, CASP1, DLK1, CLEC4D, IL24, SLIT2, HSPA1A, FSHR, IL1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression d'au moins sept gènes choisis dans le groupe constitué par : HSP90AA1, CASP1, DLK1, CLEC4D, IL-24, SLIT2, HSPA1A, FSHR, IL1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression d'au moins huit gènes choisis dans le groupe constitué par : HSP90AA1, CASP1, DLK1, CLEC4D, IL24, SLIT2, HSPA1A, FSHR, IL1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression d'au moins neuf gènes choisis dans le groupe constitué par : HSP90AA1, CASP1, DLK1, CLEC4D, IL24, SLIT2, HSPA1A, FSHR, IL1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme liquide, dans laquelle l'étape b) comprend la mesure de l'expression des gènes du groupe constitué par : HSP90AA1, CASP1, DLK1, CLEC4D, IL24, SLIT2, HSPA1A, FSHR, IL1R2 et CCND1.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins un gène choisi dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins deux gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins trois gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins quatre gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins cinq gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins six gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins sept gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins huit gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins neuf gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins dix gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins onze gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression d'au moins douze gènes choisis dans le groupe constitué par : IL-24, IL-23R, DDIT3, MMP8, DLK1, HAS1, CYR61, IL-1R2 CLEC4D, ICAM2, CASP1, MUC13 et MUC4.

Préférentiellement, ladite méthode permet d'évaluer le potentiel d'irritation oculaire d'un composé test, en particulier lorsque la mise en contact du composé test à l'étape a) est réalisée avec le composé test sous forme solide, dans laquelle l'étape b) comprend la mesure de l'expression des gènes du groupe constitué par : IL-24, IL-23R, DDIT3, MMP8, DLK1, HAS1, CYR61, IL-1R2 CLEC4D, ICAM2, CASP1, MUC13 et MUC4.

Dans un mode de réalisation particulier de la méthode, lors de l'étape a), le composé test mis en contact avec l'échantillon cornéal reconstruit in vitro est sous la forme liquide ou solide. Lorsque le composé test est liquide, il peut être utilisé pur ou dilué.

Le composé peut être dilué dans un solvant physiologiquement acceptable, tel qu'un tampon PBS (Phosphate Buffer Saline) par exemple, et est donc présent à une concentration massique comprise entre 0.1 % et 100%. Lorsque le composé est dilué il est présent à une concentration massique comprise entre 10 et 60%, plus particulièrement entre 20 et 50%, plus particulièrement entre 25 et 40% et plus particulièrement encore 30%.

L'étape c) de la méthode comprend la détermination d'un index d'irritation oculaire du composé.

Plus particulièrement, la détermination de l'index d'irritation oculaire du composé comprend l'attribution d'une valeur seuil de surexpression à chaque gène dont l'expression est mesurée.

La valeur seuil de surexpression correspond à un facteur d'augmentation de l'expression du gène lors de la mise en contact avec le composé à tester par rapport à l'expression dudit gène lors de la mise en contact avec un témoin.

De préférence, la méthode selon la présente invention comprend en outre une étape de comparaison du niveau d'expression dudit gène avec une valeur de référence. Cette valeur de référence peut servir de contrôle positif et/ou négatif.

Un contrôle positif peut par exemple être effectué en comparant le niveau d'expression dudit au moins un gène en présence du composé test avec le niveau d'expression dudit au moins un gène en présence d'un composé connu comme irritant oculaire.

Par exemple, si le niveau d'expression dudit au moins un gène en présence du composé test est supérieur ou égal au niveau d'expression dudit au moins un gène en présence d'un composé dont le potentiel irritant est connu, on pourra en conclure au potentiel irritant dudit composé.

Un contrôle négatif peut être réalisé en l'absence du composé test ou en présence d'un composé connu comme non irritant comme par exemple l'huile d'olive, le 1, 9-decadiene (CAS # 1647-16-1), le triclobarban (cas # 101-20-2 ) ou le tampon dans lequel le produit à tester est dissout ou dilué lors de son utilisation dans la méthode.

Dans le cadre de la présente invention, on pourra conclure qu'un composé test présente un potentiel irritant oculaire si une surexpression dudit gène est observée par rapport à son niveau d'expression en l'absence dudit composé test.

On entend par « surexpression », un niveau d'expression significativement plus élevé dudit gène par rapport à son niveau d'expression normal. De préférence, on entend par surexpression un niveau d'expression dans un échantillon biologique qui est supérieur à au moins 20% du niveau normal d'expression dudit gène (c'est-à-dire 1,2 fois plus), de préférence supérieur à au moins 50% du niveau normal d'expression dudit gène (c'est-à-dire 1,5 fois plus), et de manière particulièrement préférée supérieur d'au moins 90% au niveau normal d'expression dudit gène (c'est-à-dire aussi 1,9 fois plus).

Le « niveau d'expression en l'absence dudit composé test » ou « niveau normal » est le niveau d'expression dudit gène dans un échantillon contrôle correspondant potentiellement à l'échantillon biologique d'un tissu ne présentant pas de réaction de d'irritation ou, de préférence, à la moyenne du niveau d'expression dudit gène dans différents échantillons contrôle non exposé au composé test.

De préférence, l'étape b) est réalisé entre 2 et 24 heures après l'étape a), de manière encore préférée entre 4 et 18 heures après l'étape a), de manière particulièrement préférée entre 5 et 7 heures après l'étape a) et de manière encore plus préférée 6 heures après l'étape a).

Le témoin présentant un niveau d'expression normal consiste à la mise en contact de l'échantillon cornéal reconstruit in vitro avec un liquide physiologiquement acceptable non irritant, tel que par exemple le liquide dans lequel est dissout ou dilué le composé à tester, par exemple un tampon, plus particulièrement par exemple un tampon PBS.

La valeur seuil indiquant une surexpression significative du gène dont l'expression est mesurée peut être comprise en 1,1 et 10, plus particulièrement entre 1,1 et 7, plus particulièrement entre 1,4 et 6, plus particulièrement encore entre 2 et 5, plus particulièrement encore entre 2 et 4.

Comme indiqué plus avant dans un des groupes de gènes sélectionnés, dans le cas de composés test mis en contact à l'étape a) sous la forme liquide, les gènes préférés sont choisis dans le groupe comprenant au moins, ou consistant en, HSP90AA1, CASP1, DLK1, CLEC4D, IL-24, SLIT2, HSPA1A, FSHR, IL-1R2 et CCND1.

Pour HSP90AA1, la valeur seuil de surexpression est comprise entre 1,1 et 2, plus particulièrement 1,4.

Pour CASP1, la valeur seuil de surexpression est comprise entre 1,1 et 2, plus particulièrement environ 1,5.

Pour DLK1, la valeur seuil de surexpression est comprise entre 3 et 8, plus particulièrement environ 5, plus particulièrement encore 5,25.

Pour CLEC4D, la valeur seuil de surexpression est comprise entre 1,1 et 4, plus particulièrement environ 3,6.

Pour IL-24, la valeur seuil de surexpression est comprise entre 3 et 8, plus particulièrement environ 6,2.

Pour SLIT2, la valeur seuil de surexpression est comprise entre 1,1 et 2, plus particulièrement environ 1,3.

Pour HSPA1A, la valeur seuil de surexpression est comprise entre 1,1 et 5, plus particulièrement environ 3.

Pour FSHR, la valeur seuil de surexpression est comprise entre 1,1 et 4, plus particulièrement environ 2,6.

Pour IL-1R2, la valeur seuil de surexpression est comprise entre 1,1 et 2, plus particulièrement environ 1,3.

Pour CCND1, la valeur seuil de surexpression est comprise entre 1,1 et 2, plus particulièrement environ 1,5.

Comme indiqué plus avant pour un autre groupe de gènes possibles, dans le cas de composés test mis en contact à l'étape a) sous la forme solide, les gènes préférés sont choisis dans le groupe comprenant au moins, ou consistant en, IL-24, IL-23R, DDIT3, MMP8, DLK1, HAS1, CYR61, IL-1R2 CLEC4D, ICAM2, CASP1, MUC13 et MUC4.

Pour IL-24, la valeur seuil de surexpression est comprise entre 1,5 et 15, plus particulièrement entre 2 et 10, plus particulièrement 2,3.

Pour IL-24, la valeur seuil de surexpression peut aussi être comprise entre 5 et 15, plus particulièrement 10.

Pour IL-23R, la valeur seuil de surexpression est comprise entre 1,5 et 3, plus particulièrement environ 1,9.

Pour DDIT3, la valeur seuil de surexpression est comprise entre 1.5 et 3, plus particulièrement environ 5, plus particulièrement encore 2.

Pour MMP8, la valeur seuil de surexpression est comprise entre 1,1 et 2.5, plus particulièrement environ 1.4.

Pour DLK1, la valeur seuil de surexpression est comprise entre 2 et 8, plus particulièrement environ 4.

Pour HAS1, la valeur seuil de surexpression est comprise entre 1,5 et 5, plus particulièrement environ 3.

Pour CYR61, la valeur seuil de surexpression est comprise entre 1,1 et 2, plus particulièrement environ 1.3.

Pour IL-1R2, la valeur seuil de surexpression est comprise entre 1,4 et 3, plus particulièrement environ 2.

Pour CLEC4D, la valeur seuil de surexpression est comprise entre 1,5 et 3.5, plus particulièrement environ 2.8.

Pour ICAM2, la valeur seuil de surexpression est comprise entre 3 et 5, plus particulièrement environ 4.

Pour CASP1, la valeur seuil de surexpression est comprise entre 1.1 et 2, plus particulièrement environ 1.4.

Pour MUC13, la valeur seuil de surexpression est comprise entre 1.4 et 3, plus particulièrement environ 1.8.

Pour MUC4, la valeur seuil de surexpression est comprise entre 2 et 4, plus particulièrement environ 2.5.

La valeur de seuil de surexpression significative peut, pour chaque gène, être évaluée et déterminée aisément par un homme du métier.

Plus particulièrement encore, la détermination de l'index d'irritation oculaire comprend l'attribution d'une valeur de poids à chaque gène si la valeur seuil de surexpression dudit gène est atteinte suite à la mesure de l'expression.

Ainsi lorsque le seuil de surexpression significative d'un gène donné est atteint, ce gène se voit attribuer une valeur de poids qui peut être différente ou identique selon le gène. Cette valeur de poids peut prendre des valeurs discrètes ou continues, préférentiellement des valeurs discrètes échelonnées entre 1 et 10, à savoir choisies dans le groupe consistant en les valeurs 1, 2, 3, 4, 5, 6, 7, 8, 9 et 10. Plus particulièrement, les valeurs discrètes pourront être choisies dans le groupe consistant en les valeurs 1, 2, 3, 4, 5. Plus particulièrement, lesdites valeurs discrètes pourront être 1 ou 2.

A titre illustratif et sans être nullement limitatif, la valeur de poids pourra être limitée à deux valeurs, 1 ou 2, valeur qui sera attribuée au gène lorsque son seuil de surexpression significative sera atteint ou dépassé.

Le choix d'attribuer une valeur de poids plus ou moins élevée dépend de la nature du gène considéré et de son implication qui dans la réponse cellulaire au stress, qui dans la voie métabolique de l'interleukine, qui dans le processus inflammatoire, qui dans la régulation de la croissance cellulaire, qui dans la cicatrisation, qui dans le remodelage cellulaire, par exemple mais aussi de la valeur de seuil de surexpression du gène.

Aussi à titre purement illustratif et pour les gènes ici sélectionnés choisis dans le groupe comprenant, ou consistant en, HSP90AA1, CASP1, DLK1, CLEC4D, IL24, SLIT2, HSPA1A, FSHR, IL1R2 et CCND1 ; la valeur de poids pourra prendre un valeur choisie entre 1 et 2.

Pour HSP90AA1, la valeur de poids pourra être de 2.

Pour CASP1, la valeur de poids pourra être de 2.

Pour DLK1, la valeur de poids pourra être de 2.

Pour CLEC4D, la valeur de poids pourra être de 2.

Pour IL24, la valeur de poids pourra être de 1.

Pour SLIT2, la valeur de poids pourra être de 1.

Pour HSPA1A, la valeur de poids pourra être de 2.

Pour FSHR, la valeur de poids pourra être de 2.

Pour IL-1R2, la valeur de poids pourra être de 1.

Pour CCND1, la valeur de poids pourra être de 2.

De la même façon, et pour les gènes ici sélectionnés choisis dans le groupe comprenant, ou consistant en, IL-24, IL-23R, DDIT3, MMP8, DLK1, HAS1, CYR61, IL-1R2 CLEC4D, ICAM2, CASP1, MUC13 et MUC4 ; la valeur de poids pourra prendre un valeur choisie entre 1 et 4.

Pour IL-24, avec une valeur seuil surexpression de 2.3, la valeur de poids pourra être de 1.

Pour IL-24, avec une valeur seuil de surexpression de 10, la valeur de poids pourra être de 4.

Pour IL-23R, la valeur de poids pourra être de 4.

Pour DDIT3, la valeur de poids pourra être de 2.

Pour MMP8, la valeur de poids pourra être de 1.

Pour DLK1, la valeur de poids pourra être de 2.

Pour HAS1, la valeur de poids pourra être de 2.

Pour CYR61, la valeur de poids pourra être de 1.

Pour IL-1R2, la valeur de poids pourra être de 4.

Pour CLEC4D, la valeur de poids pourra être de 2.

Pour ICAM2, la valeur de poids pourra être de 4.

Pour CASP1 la valeur de poids pourra être de 2.

Pour MUC13, la valeur de poids pourra être de 3.

Pour MUC4, la valeur de poids pourra être de 4.

Plus particulièrement, l'index d'irritation oculaire est déterminé par l'addition des valeurs de poids des gènes dont l'expression dépasse la valeur seuil de surexpression.

La méthode selon l'invention peut aussi comprendre une étape de catégorisation du composé test comprenant l'attribution d'une catégorie d'irritation au composé test en fonction de la valeur de l'index d'irritation oculaire obtenu.

La méthode selon l'invention met ainsi à disposition de l'homme du métier un méthode prédictive permettant de classifier un composé selon son potentiel d'irritation oculaire en suivant la classification Européenne, à savoir non irritant, irritant réversible ou irritant non réversible.

Les résultats exposés dans la partie expérimentale de la présente demande démontrent que cet avantage de l'invention et la supériorité de cette méthode par rapport à celles existantes ressortent à l'évidence de ces résultats.

### EXEMPLES

### Matériels et Méthodes

### Modèle d'épithélium de cornée humaine reconstruit (SkinEthic^{™} HCE)

Le modèle d'épithélium de cornée humaine reconstruit, (SkinEthic^{™} HCE) a été acheté chez Episkin à Lyon. Le modèle est constitué de cellules épithéliales de cornée humaine immortalisées cultivées dans un milieu défini à la jonction air-liquide. La structure tissulaire obtenue est un épithélium multi-couche similaire à la structure tissulaire naturelle de la cornée humaine comprenant 5 -7 couches pour une surface de 0,5 cm². Les cellules polyédriques et les cellules ailées sont également présentes. Le tissu comprend aussi des ultra-structures spécifiques comme les filaments intermédiaires, les hémi-desmosomes matures et les desmosomes. La cytokératine de 65 kD (K3) a également été détectée (Nguyen et al., 2003). Les tissus sont expédiés sur une couche semi-solide de milieu de culture en agarose. A réception, les tissus sont transférés dans du milieu de maintenance (1 ml/puit) dans des plaques 6 puits et incubés dans une étuve humide à 37°C, 5% CO₂. Les tissus sont utilisés 24h plus tard.

### Produits chimiques

Tous les produits chimiques testés ont été achetés chez Sigma, France. La pureté était de plus de 87% pour tous les produits chimiques testés qui couvraient un large champ des potentiels irritants oculaires possibles.

### Préparation des produits chimiques liquides

Les produits liquides sont testés pour leur solubilité dans du tampon PBS (tampon phosphate salin) ou dans de l'huile d'olive. En résumé, 100 µl du produit à tester est mélangé à 200 µl de PBS ou d'huile d'olive. L'échantillon est mélangé sur un vortex. La turbidité et la séparation de phase éventuelle sont évaluées à l'œil. Les produits sont testés purs ou dilués à 30%. Les produits qui ne sont pas solubles dans le PBS ou l'huile d'olive ne peuvent pas être testé à 30%.

### Protocole de traitement pour les produits liquides

La procédure d'application des produits liquides sur les tissus a été optimisée de la façon suivante : les produits chimiques sont testés à deux concentrations 100% et 30%. En résumé, la surface du tissu est humidifiée par l'addition de 20 µl de PBS à 37°C et une incubation pendant 10 mn à 37°C / 5% CO₂. Les épithéliums cornéens sont ensuite traités topiquement avec 50 ± 2 µL du produit testé (ce qui correspond à 100 µL/cm2) puis incubé pendant 10 mn à température ambiante. Les tissus sont ensuite lavés avec du PBS stérile (2 X 25 ml) à 37°C. Le PBS est déposé sur le rebord de l'insert (pas directement sur le tissu) pour créer un vortex doux qui enlève le produit chimique. Les tissus sur leurs inserts sont ensuite « noyés » dans 5 ml de milieu de maintenance à température ambiante pendant 30 mn afin d'enlever le maximum le produit restant à la surface du tissu. Le milieu est ensuite retiré en tapotant doucement l'insert sur du papier absorbant et on ajoute 50 µl de milieu de maintenance à 37°C. Les inserts sont ensuite incubés pendant 6 hr à 37°C / 5% CO₂.

### Protocole de traitement pour les produits solides

La procédure d'application des produits solides (en poudre) sur les tissus a été optimisée de la façon suivante. Dans un premier temps les produits sont réduits dans la poudre la plus fine possible au moyen d'un mortier. Les produits chimiques sont testés à une seule dose. En résumé, la surface du tissu est humidifiée par l'addition de 20 µl de PBS à 37°C et une incubation pendant 10 mn à 37°C / 5% CO₂. Les épithéliums cornéens sont ensuite traités topiquement avec 30 ± 2 mg (représentant 60 mg/cm²) puis incubé pendant 30 mn à température ambiante. Les tissus sont ensuite lavés avec du PBS stérile (2 X 25 ml) à 37°C. Le PBS est déposé sur le rebord de l'insert (pas directement sur le tissu) pour créer un vortex doux qui enlève le produit chimique. Les tissus sur leurs inserts sont ensuite « noyés » dans 5 ml de milieu de maintenance à température ambiante pendant 30 mn afin d'enlever le maximum le produit restant à la surface du tissu. Le milieu est ensuite retiré en tapotant doucement l'insert sur du papier absorbant et on ajoute 50 µl de milieu de maintenance à 37°C. Les inserts sont ensuite incubés pendant 6 hr à 37°C / 5% CO₂.

### Purification des ARNs totaux

Le procédé de purification des ARNs totaux a été décrit par Cottrez et al., 2015. En résumé, les tissus de cornée sont récupérés avec des pinces et placé dans des tubes pour une congélation rapide dans de l'azote liquide. Les ARNs sont ensuite extraits par la technique du Qiazol (Qiagen, Courtaboeuf, France) avec un kit « RNeasy Mini Kit » selon les instructions du fabricant. En résumé les tissus sont placés dans 1 ml de Qiazol et homogénéisés en utilisant le TissueLyser II (Qiagen, Courtaboeuf, France) avec 2 billes d'acier. Après centrifugation le surnageant est recueilli et 0.2 ml de bromochloro propane (Sigma, France) sont ajoutés, puis l'ensemble est mélangé vigoureusement. L'homogénat est centrifugé à 12'000 g pendant 15 mn à 4 °C. La phase supérieure (phase aqueuse) est ajoutée à 600 µl d'éthanol à 70% et immédiatement mélangée par pipetage. Le mélange est transféré dans une colonne RNeasy spin placée sur un tube de collecte de 2 ml et l'ARN est recueilli selon les instructions du fabricant (Qiagen, Courtaboeuf, France) .

### Analyse par RT-PCR quantitative

La procédure de RT-PCR quantitative a été décrite par Cottrez et al., 2015. En résumé, la transcription de l'ARN total est réalisée avec 1 µg d'ARN total dans volume final de 20 µl en utilisant des « Random Primers » (Invitrogen, France) et la « SuperScript III Reverse Transcriptase » (Invitrogen, France) selon les instructions du fabricant. La RT-PCR quantitative utilise un mélange réactif de PCR : le SYBR Green real time PCR Master Mix (ROCHE, France) avec 0.4 µM de chaque primer nucléotidique dans un volume final de 25 µl. La réaction est réalisée dans un LC480 System (ROCHE, France). Le programme d'amplification comprend un cycle à 95°C pendant 1 mn, suivi de 40 cycles avec une dénaturation à 95°C pendant 15s, une phase d'hybridation et d'amplification à 60°C pendant 15s, suivi d'une phase d 'élongation finale à 72°C pendant 40s.

La quantité relative de chaque transcrit est normalisée par rapport à la quantité moyenne d'expression de 5 « housekeeping » gènes (Glucuronidase β-GUSB, vacuolar ATPase-ATP6V0E1, H2A Histone Family, Member Y- H2AFY, Glucose-6-Phosphate Dehydrogenase-G6PD and « non-POU domain containing, octamer-binding »-NONO).

### Analyse des données

### Mesure de l'expression de gènes

Le taux d'expression des gènes est mesuré par une méthode d'analyse de quantification absolue par la méthode utilisant un algorithme basé sur le maximum de la dérivée seconde développé par Roche. Le taux de surexpression relative (fold increase) est ensuite calculé par rapport aux tissus traités avec du PBS seul.

### Définition du modèle de prédiction du test d'irritation oculaire pour les liquides

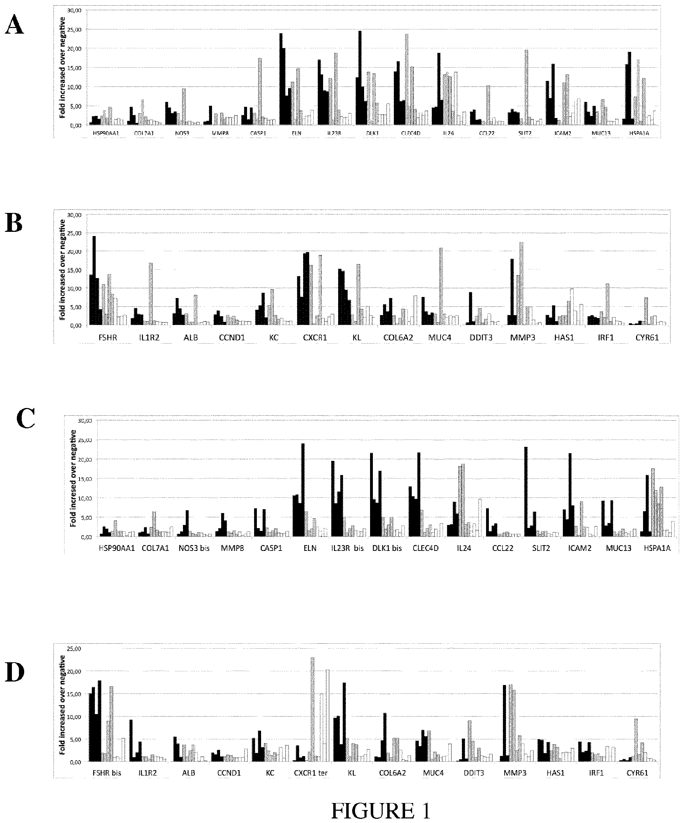

L'expression de 10 gènes (i.e.: HSP90AA1, CASP1, DLK1, CLEC4D, IL-24, SLIT2, HSPA1A, FSHR, IL-1R2, CCND1 (marqués par des étoiles dans la Figure 1) est mesurée dans les modèles d'épithélium cornéen après application soit d'une dose de 100% soit d'une dose de 30% du produit à tester comme indiqué plus haut. Les valeurs de surexpression significative a été fixé pour chacun des gènes à respectivement : 1.4; 1.5; 5.25; 3.6; 6.2; 1.3; 3; 2.6; 1.3 et 1.5. Chaque gène surexprimé reçoit ensuite une valeur fixe de respectivement : 2; 2; 2; 2; 1; 1; 2; 2; 1 ou 2. Un index d'irritation oculaire pour les substances liquides (LII : Liquid Irritation Index) est ensuite calculé par l'addition des valeurs attribuées à chaque gène surexprimé pour une valeur maximale de 17. Lorsque qu'après traitement des tissus par le produit testé la destruction tissulaire est jugée trop importante (l'ARN total collecté représente moins de 10% de la quantité d'ARN collectée dans les tissus traités par le PBS) on attribue une valeur de LII de 20.

Le modèle de prédiction et de classification fonctionne de la façon suivante. Chaque produit est testé à 100% et 30% (voir ci-dessus). Si LII ≥ 10 à 100% et 30%, le produit testé est classé "Cat I". Si LII ≥ 10 à 100% et < 10 à 30%, le produit testé est classé "Cat 2". Si LII < 10 à 100% et 30%, le produit testé est classé "No-Cat".

### Définition du modèle de prédiction du test d'irritation oculaire pour les solides

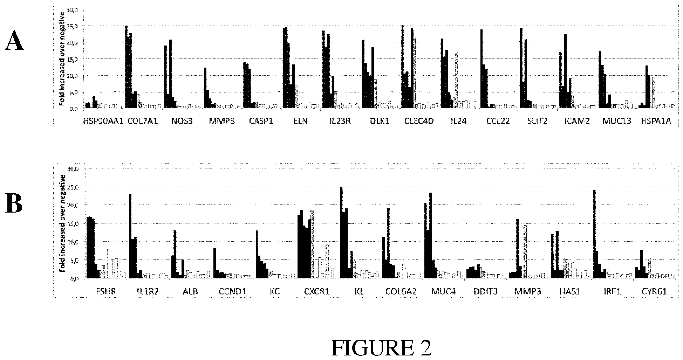

L'expression de 13 gènes (i.e.: IL-24, IL-23R, DDIT3, MMP8, DLK1, HAS1, CYR61, IL-1R2 CLEC4D, ICAM2, CASP1, MUC13, MUC4 (marqués par des étoiles dans la Figure 2)) est mesurée dans les modèles d'épithélium cornéen après application d'une dose de 60 mg/cm² du produit à tester comme indiqué plus haut. Les valeurs de surexpression significative a été fixé pour chacun des gènes à respectivement : 2.3 et 10 pour l'IL-24; puis pour chacun des autres gène dans l'ordre 1.9; 2; 1.4; 4; 3; 1.3; 2; 2.8 ; 4 ; 1.4 ; 1.8 et 2.5. Chaque gène surexprimé (ou chaque seuil dépassé pour l'IL-24) reçoit ensuite une valeur fixe de respectivement : 1; 4; 4; 2; 1; 2; 2; 1; 4 ; 2 ; 4 ; 2 ; 3 et 4. Un index d'irritation oculaire pour les substances solides (SII : Solid Irritation Index) est ensuite calculé par l'addition des valeurs attribuées à chaque gène surexprimé pour une valeur maximale de 36. Lorsque qu'après traitement des tissus par le produit testé la destruction tissulaire est jugée trop importante (l'ARN total collecté représente moins de 10% de la quantité d'ARN collectée dans les tissus traités par le PBS) on attribue une valeur de SII de 40.

Le modèle de prédiction et de classification fonctionne de la façon suivante. Chaque produit est testé à 60 mg/cm² (voir ci-dessus). Si SII ≥ 20 le produit testé est classé "Cat I". Si 10 ≤ SII < 20 le produit testé est classé "Cat 2". Si SII < 10 le produit testé est classé "No-Cat".

### Critères d'acceptabilité pour les liquides

Deux produits chimiques « contrôle » sont testés en parallèle aux produits testés. Un non-irritant : le 1, 9-decadiene (CAS # 1647-16-1) testé à 100%, et un produit de Catégorie 2 : le 2-methyl-1-pentanol (CAS #105-30-6) testé à 100% et 30%. L'essai est considéré comme valide si les valeurs de LII obtenus pour le 1,9-decadiene testé à 100% et le 2-methyl-1-pentanol testé à 30% sont inférieures à10 et si le LII pour le 2-methyl-1-pentanol testé à 100% est >10.

Chaque produit chimique est testé deux fois sur deux lots différents de tissu d'épithélium de cornée. Si ces deux analyses ont donné la même classification le test est considéré comme valide, sinon une troisième ou une quatrième analyse sont effectuées.

### Critères d'acceptabilité pour les solides

Deux produits chimiques « contrôle » sont testés en parallèle aux produits testé. Un non-irritant : le triclobarban (cas # 101-20-2 ) et un produit de Catégorie 2 : le naphthalene dione (cas # 83-56-7). L'essai est considéré comme valide si la valeur de SII obtenus pour le triclobarban est <10 et si la valeur obtenue pour le naphthalene dione est comprise entre 10 et 20.

Chaque produit chimique est testé deux fois sur deux lots différents de tissu d'épithélium de cornée. Si ces deux analyses ont donné la même classification le test est considéré comme valide, sinon une troisième ou une quatrième analyse sont effectuées.

### Analyse statistique

### Statistiques de Cooper

Les valeurs de l'analyse statistique selon Cooper (Sensibilité, Spécificité et Précision) (Cooper et al., 1979) sont calculés pour le test d'irritation oculaire en utilisant comme référence les données de la littérature pour le test de Draize (Barroso et al., 2016). Une table de contingence 2X2 avec les irritants et les non-irritants comme paramètre est construite en utilisant les résultats obtenus avec le test d'irritation oculaire. Sensibilité, Spécificité et Précision sont ensuite calculés en utilisant les préconisations de Copper . (Cooper et al., 1979).

### Analyse par matrice de confusion

Pour les statistiques utilisant les matrices de confusion et le calcul du Kappa nous avons utilisé les préconisations de Landis et Koch (Landis and Koch, 1977).

### Résultats

### Sélection des gènes biomarqueurs des mécanismes d'irritation oculaire

Pour identifier les gènes impliqués dans les mécanismes d'irritation oculaire nous avons utilisé un outil d'analyse de données développés en collaboration avec Dieng-Kuntz et coll. (Dieng-Kuntz et al., 2006). Un premier groupe de 900 gène a été sélectionné. Parmi cet important groupe de gène candidat nous avons sélectionné des gènes dont l'expression est modulée dans des échantillons prélevés à la surface de l'œil humain par un dispositif de collecte d'empreintes conjonctivales (Roy et al., 2013) . Puis nous avons utilisé une analyse de l'expression de gêne obtenu après utilisation d'un groupe de 10 produits chimiques (Table 1 #1-10) appliqué sur le modèle de tissu Skinethic HCE, associé à une recherche par data mining de la littérature existante. Cette analyse nous a permis de définir un groupe de 92 gènes impliqués dans les mécanismes qui aboutissent à une irritation oculaire (Table 2).

### Développement du modèle de prédiction du test d'irritation oculaire pour les liquides

La modification d'expression des 92 gènes que nous avons sélectionnés comme représentant de potentiels candidats biomarqueurs (Table 2) a été analysée après traitement avec 39 produits chimiques (Table 1). En utilisant un sous-groupe de 29 gènes : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61 (Figure 1) il a été possible de classer les 39 produits chimiques dans l'une des 3 catégories d'irritation oculaire mises en place par le référentiel UN-GHS. Cependant, comme certains des gènes avaient un rôle redondant et pouvaient se substituer entre eux, nous avons pu sélectionner un sous-groupe de 10 gènes permettant de mettre au point un index d'irritation pour les liquides (LII) capable de discriminer les 3 catégories d'irritants. La liste des gènes sélectionnés pour les liquides inclus : HSP90AA1, CASP1, DLK1, CLEC4D, IL-24, SLIT2, HSPA1A, FSHR, IL-1R2 and CCND1 (indiqués dans la Figure 1). Le LII a été élaboré en deux étapes. Nous avons tout d'abord sélectionné une valeur seuil pour chaque gène permettant de mettre en évidence une surexpression significative. Différentes valeurs de seuil ont été testées et les valeurs optimales retenues pour ces 10 gènes ont été respectivement de : 1.4; 1.5; 5.25; 3.6; 6.2; 1.3; 3; 2.6; 1.3 and 1.5. Nous avons ensuite attribué un poids à ces valeurs en fonction de l'importance de la surexpression du gène considéré dans les mécanismes d'irritation oculaire. Chaque gène surexprimé au-dessus de la valeur seuil recevait alors une valeur pondérée de respectivement : 2; 2; 2; 2; 1; 1; 2; 2; 1 et 2. Le LII est ensuite calculé en additionnant les valeurs de poids pour chacun des gènes surexprimés avec un maximum de 17. Lorsque le niveau de destruction tissulaire et jugé trop important (voir Matériels et Méthodes) une valeur de 20 est appliquée.

### Analyse des résultats obtenus par le test d'irritation oculaire avec les 39 produits liquides testés.

Les prédiction de classification obtenus avec les test d'irritation oculaire sur les 39 produits chimiques (Table 3) ont été comparé à ceux obtenus avec le test de Draize dans une table de contingence 2X2 pour évaluer la prédictivité du test à distinguer dans un premier temps les produits irritants (Cat 1 and Cat 2) des produits non irritants (No-Cat) (Table 4). Une spécificité, une sensibilité et une précision de 100% ont été obtenus sur ce groupe de 39 produits chimique.

Nous avons ensuite analysé une matrice de confusion contenant 3 classes (Cat 1, Cat 2 and No-Cat, voir Table 5) pour calculer les performances du test à classer les produits selon les normes UN-GHS. Une précision pour l'utilisateur de 91.66%, 93.33% and 100% a été obtenu pour respectivement les produits classé Cat 1, Cat 2 et No-Cat. Avec une précision générale de 95% et un kappa hautement significatif de 0.923.

Ces résultats montrent que le test d'irritation oculaire est très efficace pour classer le potentiel d'irritation oculaire des produits chimiques liquides dans les 3 classes définies par le référentiel UN-GHS.

### Développement du modèle de prédiction du test d'irritation oculaire pour les solides

Pour les solides nous avons testé 15 produits chimiques (Table 6). En utilisant le même groupe de 29 gènes : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61 (Figure 2) il a été possible de classer les 15 produits chimiques dans l'une des 3 catégories d'irritation oculaire mises en place par le référentiel UN-GHS. Cependant, comme certains des gènes avaient un rôle redondant et pouvaient se substituer entre eux, nous avons pu sélectionner un sous-groupe de 13 gènes permettant de mettre au point un index d'irritation pour les solides (SII) capable de discriminer les 3 catégories d'irritants. La liste des gènes sélectionnés pour les solides inclut : (IL-24, IL-23R, DDIT3, MMP8, DLK1, HAS1, CYR61, IL-1R2, CLEC4D, ICAM2, CASP1, MUC13, MUC4. Figure 2). Le SII a été élaboré en deux étapes. Nous avons tout d'abord sélectionné une valeur seuil pour chaque gène permettant de mettre en évidence une surexpression significative. Différentes valeurs de seuil ont été testées et les valeurs optimales retenues pour ces 13 gènes ont été respectivement de 2.3 et 10 pour l'IL-24; puis pour chacun des autres gènes dans l'ordre 1.9; 2; 1.4; 4; 3; 1.3; 2; 2.8 ; 4 ; 1.4 ; 1.8 et 2.5. Nous avons ensuite attribué un poids à ces valeurs en fonction de l'importance de la surexpression du gène considéré dans les mécanismes d'irritation oculaire. Chaque gène surexprimé au-dessus de la valeur seuil recevait alors une valeur pondérée de respectivement : 1; 4; 4; 2; 1; 2; 2; 1; 4 ; 2 ; 4 ; 2 ; 3 et 4. Le SII est ensuite calculé en additionnant les valeurs de poids pour chacun des gènes surexprimés avec un maximum de 36. Lorsque le niveau de destruction tissulaire et jugé trop important (voir Matériels et Méthodes) une valeur de 40 est appliquée.

### Analyse des résultats obtenus par le test d'irritation oculaire avec les 15 produits solides testés.

Les prédiction de classification obtenus avec les test d'irritation oculaire sur les 15 produits chimiques solides (Table 7) ont été comparé à ceux obtenus avec le test de Draize dans une table de contingence 2X2 pour évaluer la prédictivité du test à distinguer dans un premier temps les produits irritants (Cat 1 and Cat 2) des produits non irritants (No-Cat) (Table 8). Une spécificité, une sensibilité et une précision de 100% ont été obtenues sur ce groupe de 15 produits chimique.

Nous avons ensuite analysé une matrice de confusion contenant 3 classes (Cat 1, Cat 2 and No-Cat, voir Table 9) pour calculer les performances du test à classer les produits selon les normes UN-GHS. Une précision pour l'utilisateur de 100% a été obtenue pour les produits classés Cat 1, Cat 2 et No-Cat. Avec une précision générale de 100% et un kappa parfaitement significatif de 1.

Ces résultats montrent que le test d'irritation oculaire est très efficace pour classer le potentiel d'irritation oculaire des produits chimiques dans les 3 classes définies par le référentiel UN-GHS.

### Légende des figures

**Figure 1** **A, B, C, D:** Analyse d'expression génique dans le tissu cornéal humain reconstruit en 3D traité avec différents produits chimiques liquides irritants et non irritant irritants.

La valeur du taux de surexpression (fold increase) pour les 29 gènes indiqués a été représentée pour 4 produits chimiques de catégorie 1 (barre noires, dans l'ordre de gauche à droite acide lactique, methyl thioglycolate, sodium lauryl sulfate (15%), Benzalkonium choride (10%)), 4 produits chimiques de catégorie 2 (barres grises, dans l'ordre de gauche à droite alpha hexyl cinnamaldehyde, acetone, methyl ethyl ketone, 3-chloro propane nitrile) et 4 produits non classés (No category, barres blanches dans l'ordre de gauche à droite : 1,9 decadiene, Glycerol, Tween 20, 2,4 pentanediol). Chaque produit chimique a été appliqué à 100% (A et B) ou dilué à 30% (C et D). Les gènes utilisés pour l'exemple sont HSP90AA1, CASP1, DLK1, CLEC4D, IL-24, SLIT2, HSPA1A, FSHR, IL-1R2 and CCND1.

**Figure 2** **A et B :** Analyse d'expression géniques dans le tissu cornéal humain reconstruit en 3D traité avec différents produits chimiques solides irritants et non irritant irritants.

La valeur du taux de surexpression (fold increase) pour les 29 gènes indiqués a été représentée pour 5 produits chimiques de catégorie 1 (barre noires, dans l'ordre de gauche à droite, 2 hydroxyisobutyric acide, Hydrochloride de Promethazine, Sodium oxalate, 2,5-dimethyl heanediol, 1-naphthalene acetic acide), 2 produits chimiques de catégorie 2 (barres grises dans l'ordre de gauche à droite, Naphthalene diol, Camphene) et 8 produits non classés (No category, barres blanches dans l'ordre de gauche à droite Triclocarban, Methylenebis benzotriazol tetramethylbutyl phenol, Pyrimetranyl, Myristil myristate, 4,4' Methylene bis-(2,6-di-tert-butylphenol), 4-bromophenetol, Potassium tetrafluoroborate). Chaque produit chimique a été appliqué à pur en poudre (30 ± 2 mg (représentant 60 mg/cm²)) . Les gènes sélectionnés pour l'exemple sont IL-24, IL-23R, DDIT3, MMP8, DLK1, HAS1, CYR61, IL-1R2 CLEC4D, ICAM2, CASP1, MUC13, MUC4.

**Table 1 : Liste des produits liquides étudiés**

| **N°** | **Chemical** | **CAS RN** | **UN GHS** |
|---|---|---|---|
| 1 | Triton X100 10% | 9002-93-1 | Cat 1 |
| 2 | Triton X100 5% | 9002-93-1 | Cat 2A |
| 3 | Triton X100 1% | 9002-93-1 | No Cat |
| 4 | Sodium lauryl sulphate 3% | 151-21-3 | No Cat |
| 5 | Methyl ethyl ketone | 78-93-3 | Cat 2A |
| 6 | Acetone 100% | 67-64-1 | Cat 2A |
| 7 | Lactic acid | 50-21-5 | Cat 1 |
| 8 | Chlorexhidine 50% | 55-56-1 | Cat 1 |
| 9 | Benzalkonium chloride 5% | 8001-54-5 | Cat 1 |
| 10 | Hexadecyltrimethylammonium bromide 10% | 57-09-0 | Cat 1 |
| 11 | Triton X-100 10% | 9002-93-1 | Cat 1 |
| 12 | Benzethonium chloride 10% | 121-54-0 | Cat 1 |
| 13 | Methyl thioglycolate | 2365-48-2 | Cat 1 |
| 14 | Diethylaminopropionitrile | 5351-04-2 | Cat 1 |
| 15 | Tetraethylene glycol diacrylate | 17831-71-9 | Cat 1 |
| 16 | 1-Chloroctan-8-ol | 23144-52-7 | Cat 1 |
| 17 | [3-(2-Aminoethylamino)propyl]trimethoxysilane | 1760-24-3 | cat 1 |
| 18 | Sodium hydroxyde 0,3% | 1310-73-2 | Cat 2A |
| 19 | 2, 6-dichlorobenzoyl chloride | 4659-45-4 | Cat 2A |
| 20 | Gammabutyrolactone | 96-48-0 | Cat 2A |
| 21 | Allyl alcohol | 107-18-6 | Cat 2A |
| 22 | Chlorhexidine gluconate (20%) | 18472-51-0 | Cat 2A |
| 23 | Propasol Solvent P | 1569-01-3 | Cat 2A |
| 24 | 2-methyl-1-pentanol | 105-30-6 | Cat 2B |
| 25 | iso-Butanal | 78-84-2 | Cat 2B |
| 26 | 3-Chloropropionitrile | 542-76-7 | Cat 2B |
| 27 | Ethyl-2-methylacetoacetate | 609-14-3 | Cat 2B |
| 28 | Glycolic acid (10%) | 79-14-1 | Cat 2B |
| 29 | diethyl toluamide | 134-62-3 | Cat 2B |
| 30 | Glycerol | 56-81-5 | No Cat |
| 31 | Tween 20 | 9005-64-5 | No Cat |
| 32 | octyltrimethoxysilane ( SILAN 108) | 3069-40-7 | No Cat |
| 33 | 1, 9-decadiene | 1647-16-1 | No Cat |
| 34 | 2,4-Pentanediol | 625-69-4 | No Cat |
| 35 | 2-Ethoxyethyl methacrylate | 2370-63-0 | No Cat |
| 36 | dipropyl disulphide | 629-19-6 | No Cat |
| 37 | n-Hexyl bromide | 111-25-1 | No Cat |
| 38 | Polyoxyethylene hydrogenated castor oil (Kolliphor) | 61788-85-0 | No Cat |
| 39 | 1-ethyl-3-methylimidazolium ethylsulphate | 342573-75-5 | No Cat |

**Table 2 : Liste des gènes analysés par RT-PCR**

| **SYMBOLE** | **DESCRIPTION** | **Fonction** | **REFSEQ** |
|---|---|---|---|
| ALB | Albumin | protéine majeure de la cornée | NM_000477 |
| ATP6V0E 1 | ATPase, H+ transporting, lysosomal 9kDa, V0 subunit e1 | Impliqué dans phosphorylation oxydative et phagosome | NM_003945.3 |
| B4GALT6 | UDP-Gal:betaGlcNA c beta 1,4-galactosyltra nsferase, polypeptide 6 | Requise pour la biosynthèse de glycophospholipides. plus exprimé dans la cornée que les autres membres de la famille. | NM_004775 |
| CASP1 | caspase 1, apoptosis-related | Activation de IL-1. | NM_033292 |
| | cysteine peptidase (interleukin 1, beta, convertase) | | |
| CCL22 | chemokine (C-C motif) liGand 22 | Ligand de CCR4 ; impliqué dans le transit des cellules T. | NM_002990.3 |
| CCND1 | cyclin D1 | Régulateur positif du cycle cellulaire | NM_053056 |
| CCNF | cyclin F | Régulateur du cycle cellulaire | NM_001761.2 |
| CCS | copper chaperone for superoxide dismutase | Delivre le Cuivre à la zinc superoxide dismutase Cuivre dépendante (SOD1) | NM_005125 |
| CLEC4D | C-type lectin domain family 4, member D | Récepteur endocytaire. Impliqué dans le traitement des antigènes. | NM_080387.4 |
| COL17A1 | collagen, type XVII, alpha 1 | Joue un rôle dans l'attachement des kératinocytes basals à la membrane basale. | NM_000494 |
| COL6A2 | collagen type | Joue le rôle de protéine de liaison | NM_001849.3 |
| | VI alpha 2 | cellulaire | |
| COL7A1 | collagen, type VII, alpha 1 | Protéine de membrane de l'épithélium squameux stratifié. | NM_000094 |
| COL8A2 | collagen, type VIII, alpha 2 | Composant majeur de la membrane de Descemet des cellules endothéliales cornéales. Marqueurs des cellules endothéliales cornéales humaines. | NM_005202.2 |
| CRYAB | crystallin, alpha B | Susceptible de contribuer à la transparence et à l'index de réfraction de la lentille | NM_001885 |
| CSF2 | colony stimulatinG factor 2 (Granulocyte-macrophaGe) | Facteur de croissance des cellules hématopoïétiques | NM_000758.2 |
| CTGF | connective tissue Growth factor | Tissu connectif micro attractif majeur sécrété par les cellules endothéliales | NM_001901.2 |
| | | vasculaires. | |
| CTSZ | cathepsin Z | Cystéine protéinase impliquée dans la dégradation protéique lysosomale et extra-cellulaire | NM_001336.2 |
| CXCL1 | chemokine (C-X-C motif) liGand 1 (melanoma Growth stimulatinG activity, alpha) | Dispose d'une activité chimiotactique pour les neutrophiles. | NM_001511.1 |
| CXCL9 | chemokine (C-X-C motif) liGand 9 | Dispose d'une activité chimiotactique pour les cellules T. se lie au CXCR3 | NM_002416.1 |
| CXCR1 | chemokine (C-X-C motif) receptor 1 | Récepteur de l'interleukin-8, facteur chimiotactique des neutrophiles. | NM_000634.2 |
| CYR61 | cysteine-rich, angiogenic inducer, 61 | Impliqué dans la néo vascularisation de la cornée | NM_001554.4 |
| CYYR1 | cysteine/tyro | Marqueur spécifique | NM_052954.2 |
| | sine-rich 1 | des cellules endothéliales cornéales humaines. | |
| DDIT3 | DNA-damaGe-inducible transcript 3 | Marqueur d'apoptose | NM_004083.4 |
| DEFB1 | defensin, beta 1 | Impliqué dans la défense mucosale contre l'agression | NM_005218 |
| DLK1 | delta-like 1 homolog (Drosophila) | Ligand non canonique impliqué dans le développement des tissus | NM_003836 |
| DUOX2 | Dual oxidase 2 | Membre de la famille des NADPH oxydase | NM_014080 |
| DUSP6 | dual specificity phosphatase 6 | Cible la famille des ERK et joue une rôle dans la prolifération des cellules cornéales. | NM_001946.2 |
| ELN | elastin | Protéine majeur de structure des tissus. | NM_000501.2 |
| ESR1 | estrogen receptor 1 | Récepteur nucléaire hormonal. Affecte la prolifération cellulaire et la différentiation des | NM_000125 |
| | | tissus cible. | |
| FBN1 | fibrillin 1 | Fibrillines sont des composants structuraux parfois associés à l'élastine. | NM_000138 |
| FBN2 | fibrillin 2 | Fibrillines sont des composants structuraux parfois associés à l'élastine | NM_001999 |
| FDXR | ferredoxin reductase | flavoprotéine mitochondriale qui initie le transport d'électron pour les cytochromes P450 recevant des électrons du NADPH | NM_004110.3 |
| FGF2 | fibroblast growth factor 2 (basic) | Facteur de croissance et agent angiogénique. | NM_002006 |
| FGFR1 | fibroblast growth factor receptor 1 | Récepteur pour facteurs de croissance de fibroblaste (FGF1 et FGF2) | NM_015850 |
| FOS | FBJ murine osteosarcoma viral | Phosphoprotéine nucléaire formant un complexe fort mais | NM_005252 |
| | oncogene homolog | non covalent avec le facteur de transcription JUN/AP-1. | |
| FOXO1 | forkhead box O1 | Facteur de Transcription impliqué dans les voies métaboliques de l'insuline | NM_002015 |
| FSHR | follicle stimulating hormone receptor | Receptor for follicle-stimulating hormone (FSH). | NM_000145 |
| FST | follistatin | Inhibiteur de FSH | NM_013409.2 |
| G6PD | Glucose-6-phosphate dehydroGenase | Produit des pentoses pour la synthèse d'acides nucléiques et principal producteur de pouvoir réducteur NADPH | NM_000402 |
| GAA | Glucosidase, alpha; acid | Essentiel pour la dégradation du glycogène en glucose dans les lysosomes. | NM_000152.3 |
| GSTT1 | Glutathione S-transferase theta 1 | Conjugaison du glutathionne réduit avec de nombreux composes | NM_000853.2 |
| | | électrophiles exogènes et endogènes. | |
| GUSB | glucuronidase , beta | Rôle important dans la dégradation des dermatan sulfates et des keratan sulfates. | NM_000181 |
| H2AFY | H2A histone family, member Y | Variant de l'histone H2A remplaçant la H2A conventionnelle dans un sous-groupe de nucléosomes là où il réprime la transcription. | NM_138610.2 |
| HAS1 | hyaluronan synthase 1 | Joue un rôle dans la synthèse de hyluronan et d'acide hyaluronique. | NM_001523 |
| HSP90AA 1 | heat shock protein 90kDa alpha (cytosolic), class A member 1 | Chaperonne moléculaire qui promeut la maturation, le maintien, la structure et la régulation de protéines cible spécifiques | NM_005348 |
| | | impliquées dans le contrôle du cycle cellulaire et le signal de transduction | |
| HSPA1A | heat shock 70kDa protein 1A | Hsp70s stabilise les protéines contre l'agrégation et aide au repliement des nouveaux peptides traduits dans le cytosol et dans les organelles. | NM_005345 |
| ICAM2 | intercellular adhesion molecule 2 | ICAM sont les ligands pour l'adhésion des protéines LFA-1 aux leucocytes. | NM_001099789 .1 |
| IER3 | immediate early response 3 | Peut jouer un rôle dans le signal ERK en inhibant la déphosphorylation de ERK par la phosphatase PP2A-PPP2R5C holoenzyme | NM_003897.3 |
| IGFBP2 | insulin-like Growth factor bindinG protein 2, 36kDa | Inhibe le développement et la croissance médiés par l'IGF. | NM_000597.2 |
| IL-17C | interleukin 17C | Stimule la libération du facteur de nécrose tumoral alpha et IL-1beta de la lignée cellulaire monocytique THO-1. | NM_013278 |
| IL-1 R2 | interleukin 1 receptor, type II | Récepteur de l'interleukine-1 alpha (IL-1A), beta (IL-1B) et de la protéine antagoniste du récepteur interleukine-a (IL-1ra). | NM_173343.1 |
| IL-23R | interleukin 23 receptor | S'associe à IL12RB1 pour former le récepteur interleukine-23. | NM_144701.2 |
| IL-24 | interleukin 24 | Rôle dans la cicatrisation | NM_181339.1 |
| IRF1 | interferon regulatory factor 1 | Se lie spécifiquement à la région amont régulatrice des gènes de type I IFN et IFN du CMH et active ces gènes. | NM_002198 |
| ITGA6 | integrin, | Integrine alpha-6/beta-4 est un | NM_000210.2 |
| | alpha 6 | récepteur de laminine dans les cellules épithéliales et joue un rôle critique structural dans l'hémidesmosome. | |
| ITGA7 | integrin, alpha 7 | Integrine alpha-7/beta-1 est le récepteur primaire de laminine au niveau des myoblastes squelettiques et des myofibres adultes. | NM_002206 |
| JUN | jun proto-oncogene | Facteur de transcription qui reconnait et se lie au motif heptamer 5'-TGA[CG]TCA-3' | NM_002228 |
| KL | klotho | Impliqué dans les processus de vieillissement | NM_004795 |
| KRT1 | keratin 1 | membre de la famille des kératines | NM_006121.3 |
| KRT14 | keratin 14 | membre de la famille des kératines | NM_000526.4 |
| KRT15 | keratin 15 | membre de la famille des kératines | NM_002275.3 |
| KRT17 | keratin 17 | membre de la famille des kératines | NM_000422.2 |
| KRT19 | keratin 19 | membre de la famille des kératines | NM_002276.4 |
| KRT3 | keratin 3 | membre de la famille des kératines | NM_057088.2 |
| KRT5 | keratin 5 | membre de la famille des kératines | NM_000424 |
| KRT9 | keratin 9 | membre de la famille des kératines | NM_000226.2 |
| MMP10 | matrix metallopeptid ase 10 (stromelysin 2) | Peut dégrader la fibronectine, les gélatines de type I, III, IV et V. | NM_002425.1 |
| MMP13 | matrix metallopeptid ase 13 (collagenase) | Dégrade le collagène de type I. pas d'action sur gélatine ou caséine. | NM_002127.2 |
| MMP3 | matrix metallopeptid ase 3 (stromelysin 1, proGelatinase ) | Dégrade fibronectine, laminine, gélatines de type I, III, IV, and V; collagènes III, IV, X, and IX, et protéoglycanes du cartilage. | NM_002422.3 |
| MMP8 | matrix | Dégrade les | NM_002424.2 |
| | metallopeptid ase 8 (neutrophil collaGenase) | collagènes fibrillaires de type I, II et III. | |
| MSN | moesin | Impliqué dans les connexions des structures cytosquelettiques majeurs de la membrane plasmatique. | NM_002444.2 |
| MSRA | Methionine sulfoxide reductase A | Fonctionne comme enzyme de réparation des protéines inactivées par l'oxydation. | NM_012331 |
| MUC1 | mucin 1, cell surface associated | Exprimée par cellules apicales et joue rôle dans le film lacrymal | NM_001204285 .1 |
| MUC13 | mucin 13, cell surface associated | Exprimée par cellules apicales et joue rôle dans le film lacrymal | NM_033049 |
| MUC16 | mucin 16, cell surface associated | Exprimée par cellules apicales et joue rôle dans le film lacrymal | NM_024690 |
| MUC4 | mucin 4, cell surface associated | Exprimée par cellules apicales et joue rôle dans le film lacrymal | NM_018406 |
| MYD88 | myeloid differentiati on primary response gene (88) | Protéine impliquée dans les voies de signalisation du récepteur Tool-like et IL-1 | NM_001172569 .1\| |
| NONO | non-POU domain containing, octamer-binding | Protéines de liaison à l'AND et ARN ; impliquée dans de nombreux processus nucléaires. | NM_007363 |
| NOS3 | nitric oxide synthase 3 (endothelial cell) | Produit du NO impliqué dans la relaxation vasculaire du muscle lisse. | NM_000603 |
| OCLN | occludin | Rôle dans la formation et la régulation des jonctions forte , barrière paracellulaire de perméabilité. | NM_002538.3 |
| PER1 | period homolog 1 | Composant du mécanisme circadien | NM_002616 |
| | (Drosophila) | essentiel pour la génération des rythmes circadiens. | |
| RAD23A | RAD23 homolog A (S. cerevisiae) | Récepteur multiubiquitine impliqué dans la modulation de la dégradation par protéasome | NM_005053 |
| S100A4 | S100 calcium bindinG protein A4 | - | NM_002961.2 |
| SIRT6 | sirtuin 6 | Déacétylase NAD-dépendante. Module l'acétylation des histones H3 au niveau de la chromatine télomérique Durant la phase S du cycle cellulaire. | NM_016539 |
| SIRT7 | sirtuin 7 | Deacetylase NAD-dépendante. Requise pour restaurer la transcription de l'ARNr à la sortie de la mitose. | NM_016538 |
| SLC4A11 | solute carrier family 4, | Transporteur jouant un rôle important dans le | NM_001174090 .1 |
| | sodium borate transporter, member 11 | transport de fluide médié par le sodium dans différents organes. | |
| | | Prévient les modifications morphologiques graves causées par une augmentation des concentrations de NaCl dans le stroma. | |
| SLIT2 | slit homolog 2 (Drosophila) | Agit comme guide moléculaire dans la migration cellulaire et semble modulé par l'interaction avec récepteurs homologues. | NM_004787 |
| SNN | stannin | Joue un rôle dans les effets toxiques des organotines. | NM_003498 |
| STK25 | serine/threon ine kinase 25 | Serine/thréonine kinase oxydante activée par le stress susceptible de jouer un rôle dans la réponse au stress environnemental. | NM_006374.3 |
| | | . | |
| THBS1 | thrombospondi n 1 | Glycoprotein adhésive médiant les interactions cellules-cellules et cellules-matrices. Se lie à l'Héparine. | NM_003246 |
| TNFAIP3 | tumor necrosis factor, alpha-induced protein 3 | Enzyme d'édition dépendante de l'ubiquitine contenant des activités ubiquitine-ligase et deubiquitinase. | NM_006290.2 |
| TPSAB1 | tryptase alpha/beta 1 | La tryptase est une protéase majeure présente dans les cellules mastocytes et sécrétée lors de la réponse par la dégranulation/activa tion de ce type de cellules. | NM_003294.3 |

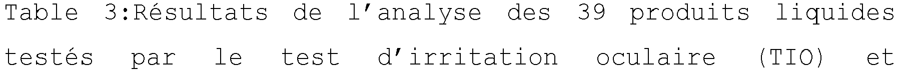

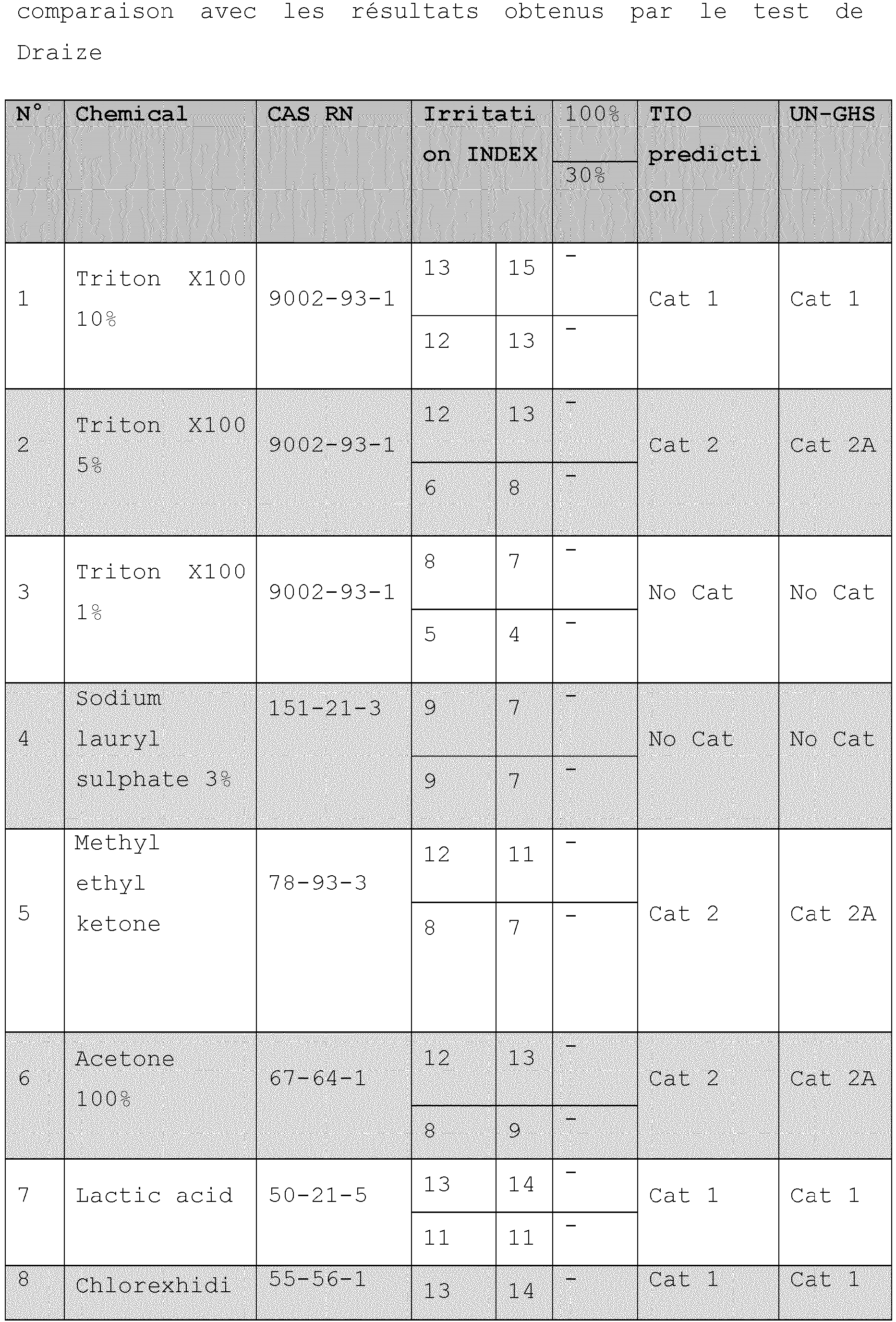

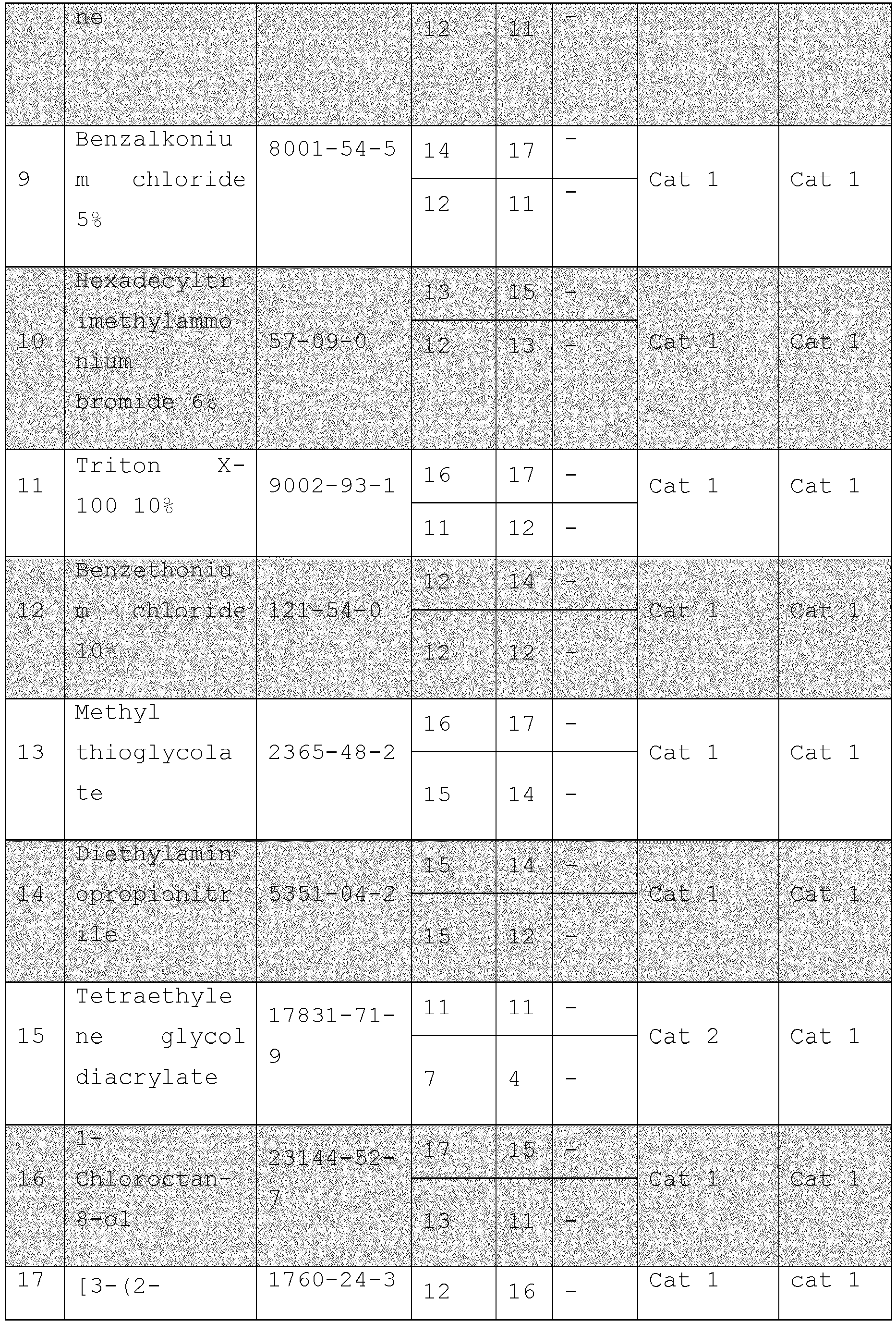

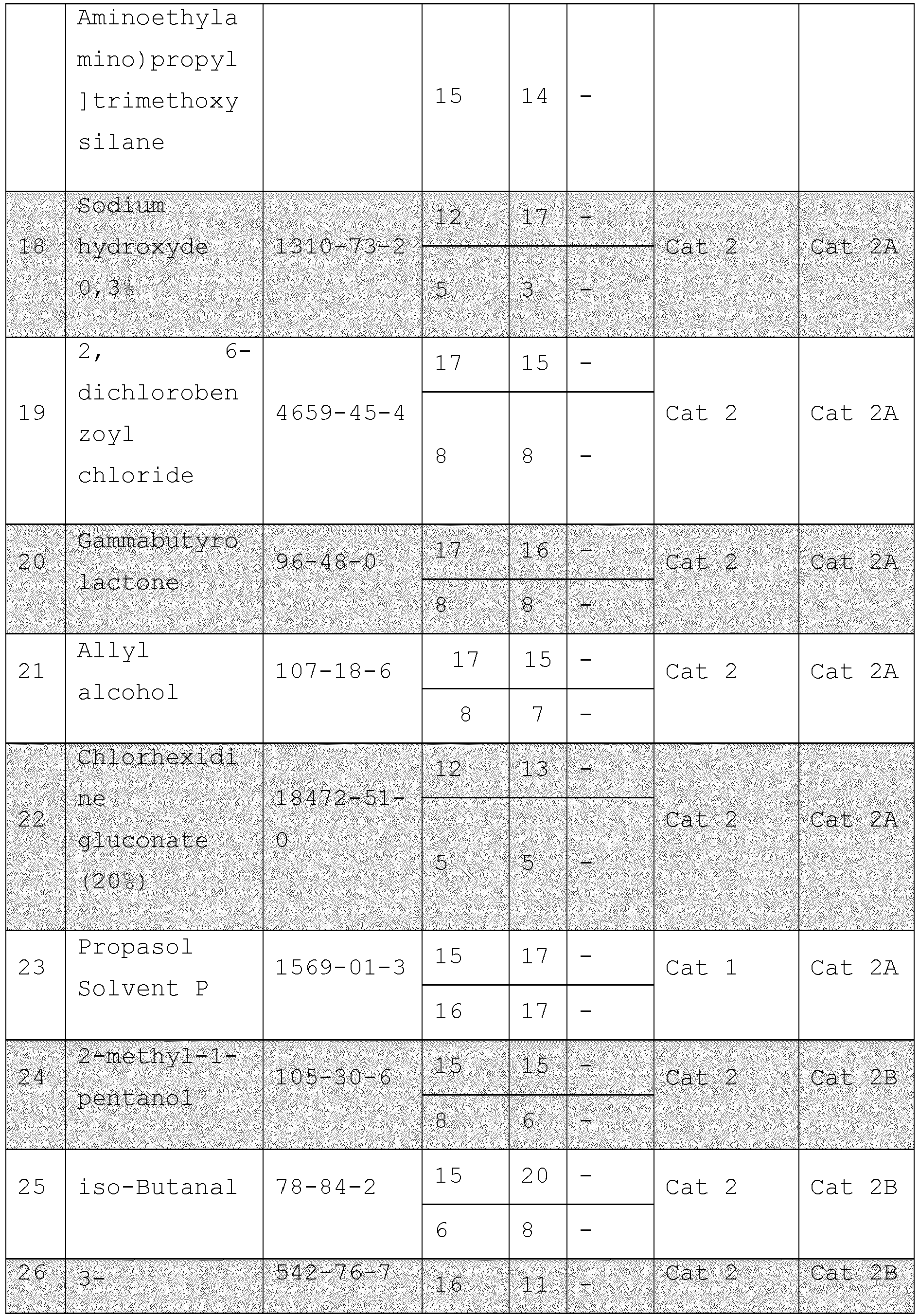

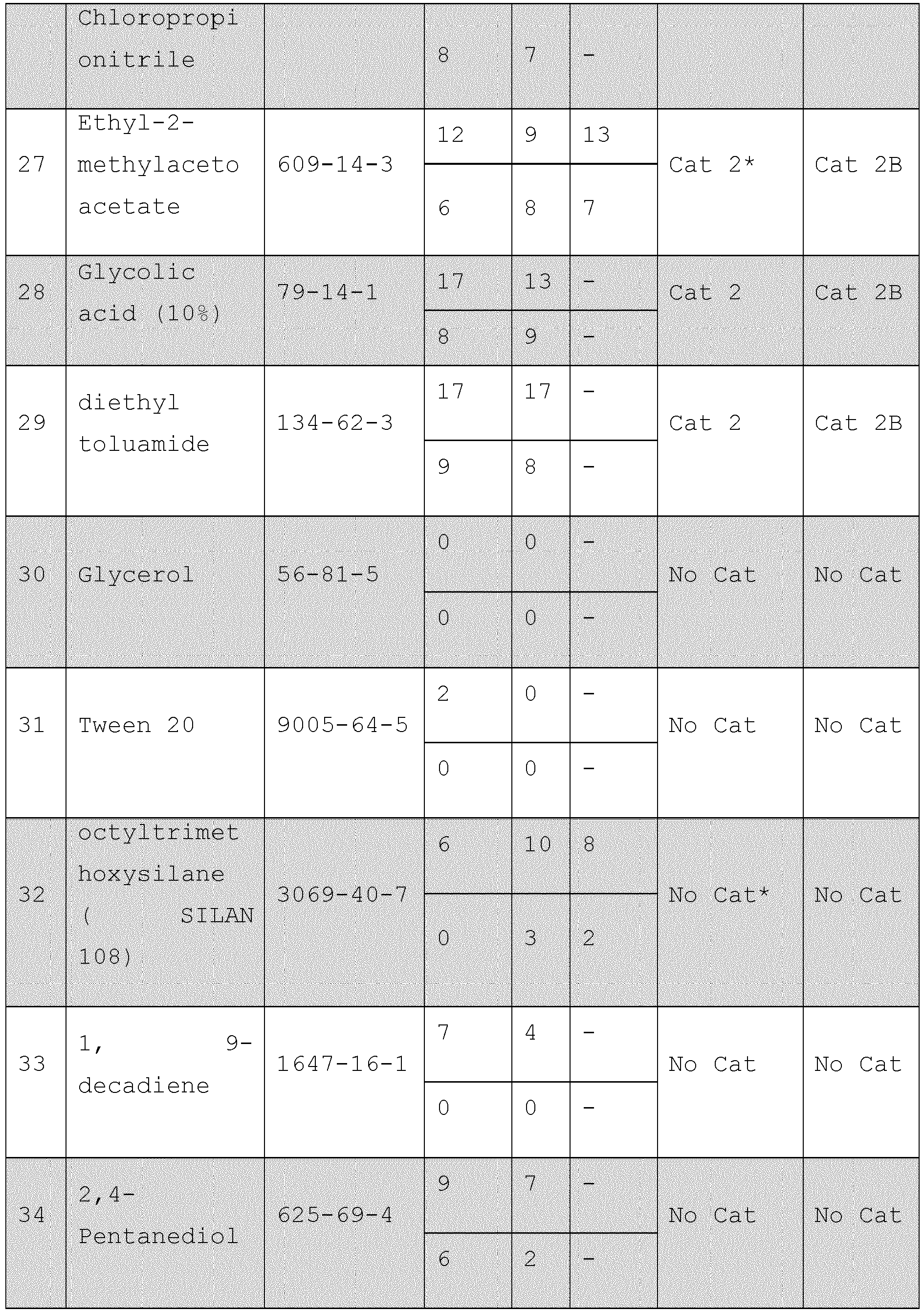

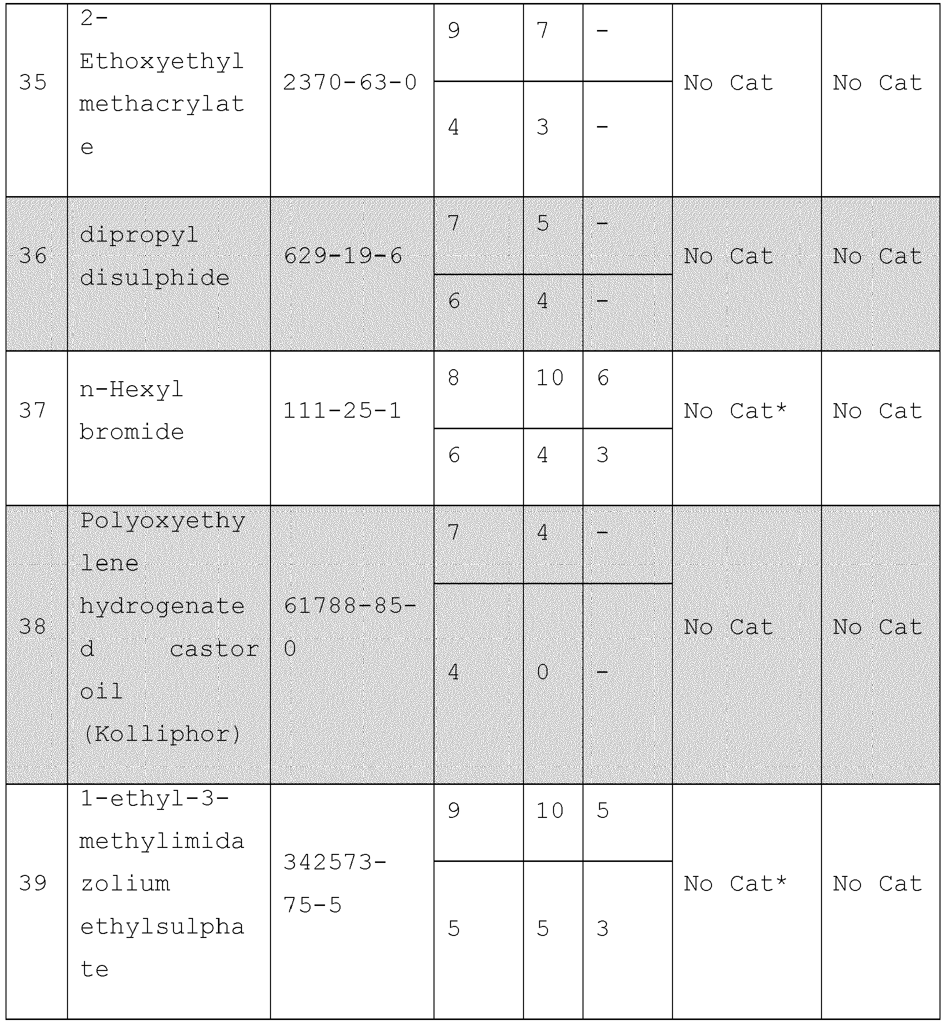

**Table 4: Mesure de la capacité de prédiction du test d'irritation oculaire pour différencier les irritants oculaires (classés comme Cat 1 ou Cat 2) des non irritants (no Cat) sur un set de 39 produits chimiques**

| DRAIZE | Classés | No Categorie |
|---|---|---|
| Classés (n) | 27 | 0 |
| No Categorie (n) | 0 | 12 |
| Total (n) | 27 | 12 |
| Sensitivité (%) | 100 | |
| Specificité (%) | 100 | |
| Précision (%) | 100 | |

**Table 5 : Analyse par matrice de confusion de la capacité de prédiction du test d'irritation oculaire à séparer entre 3 classes d'irritation (Cat 1, Cat 2 and no Cat) selon les recommandations UN-GHS.**

| | | Test d'irritation oculaire | | | | |
|---|---|---|---|---|---|---|
| | | Cat 1 | Cat 2 | No Cat | Classification Overall | Producer accuracy (Precision) |
| UNGHS | Cat 1 | 11 | 1 | | 12 | 91.66% |
| | Cat 2 | 1 | 14 | | 15 | 93.33% |
| | No Cat | | | 12 | 12 | 100% |
| | Truth Overall | 12 | 15 | 12 | 39 | |
| | User accuracy (Recall) | 91.6 6% | 93.3 3% | 100 % | | |
| Overall Accuracy | 95% | | | | | |
| Kappa | 0 .923 | | | | | |

**Table 6 : Liste des produits chimiques solides étudiés**

| **CAS Number** | **Nom CHIMIQUE** | **GHS Classe** |
|---|---|---|
| 594-61-6 | alpha-Hydroxyisobutyric acid [2-Hydroxyisobutyric acid] | Cat 1 |
| 58-33-3 | Promethazine Hydrochloride | Cat 1 |
| 79-92-5 | Camphene | Cat 2B |
| 62-76-0 | Sodium oxalate | Cat 1 |
| 110-03-2 | 2,5-Dimethylhexanediol | Cat 1 |
| 86-87-3 | 1-Naphthalene acetic acid | Cat 1 |
| 83-56-7 | 1,5-naphthalenediol INCI name: 1,5-NAPHTHALENE DIOL | Cat 2A |
| 101-20-2 | 1-(4-chlorophenyl)-3-(3,4-dichlorophenyl) urea INCI name:TRICLOCARBAN | no cat |
| 103597-45-1 | 2,2'-methylene-bis-(6-(2H-benzotriazol-2-yl)-4-(1,1,3,3-tetramethylbutyl)phenol) INCI name: METHYLENE BIS-BENZOTRIAZOLYL TETRAMETHYLBUTYLPHENOL | no cat |
| 53112-28-0 | 2-anilino-4, 6-dimethylpyrimidine common name: Pyrimethanil | no cat |
| 3234-85-3 | tetradecyl tetradecanoate INCI name: Myristyl myristate | No cat |
| 118-82-1 | 4,4'-Methylene bis-(2,6-di-tert-butylphenol) | No cat |
| 589-10-6 | 4-Bromophenetol | No cat |
| 14075-53-7 | Potassium tetrafluoroborate | No cat |
| 21645-51-2 | Aluminum hydroxyde | No cat |

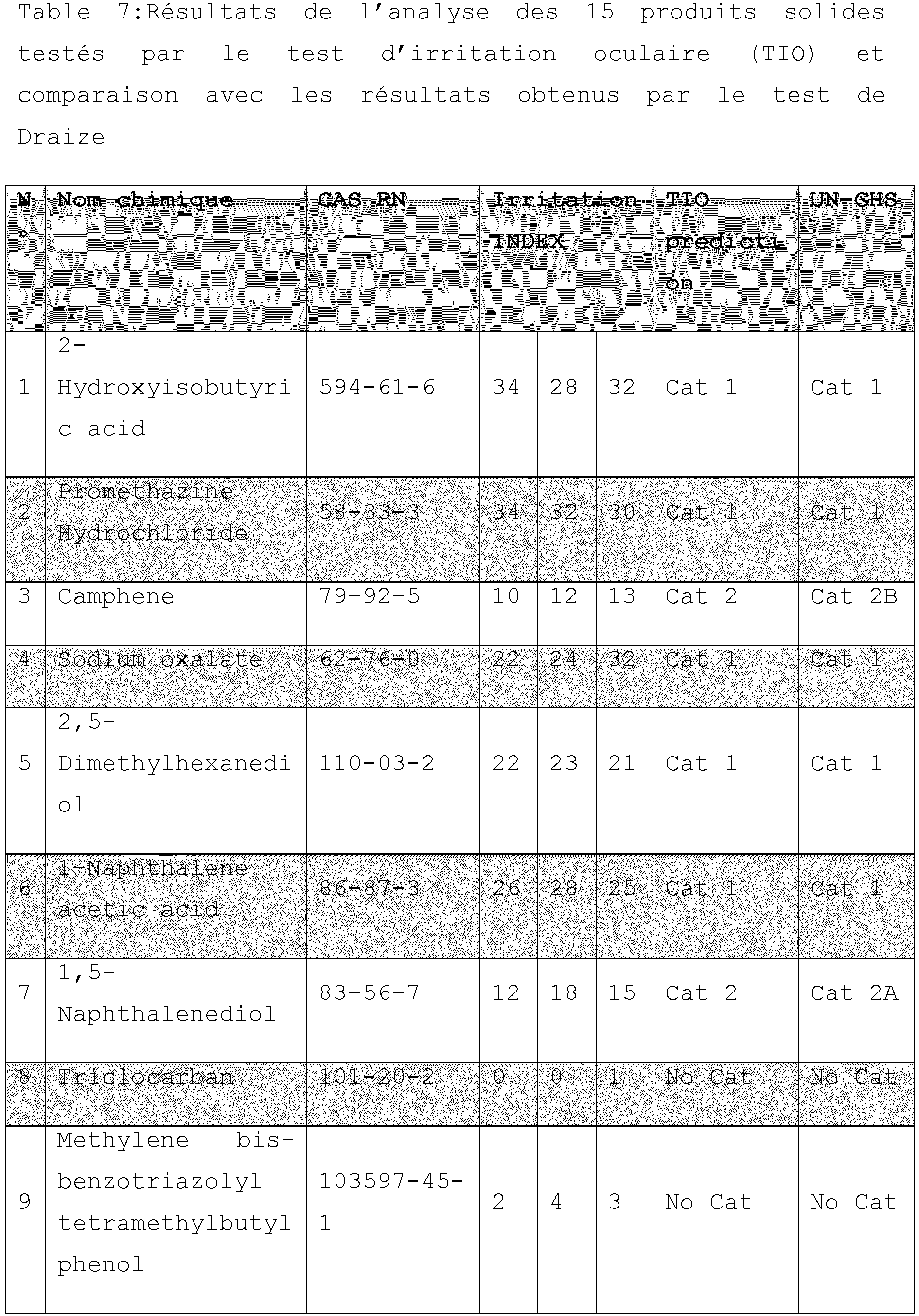

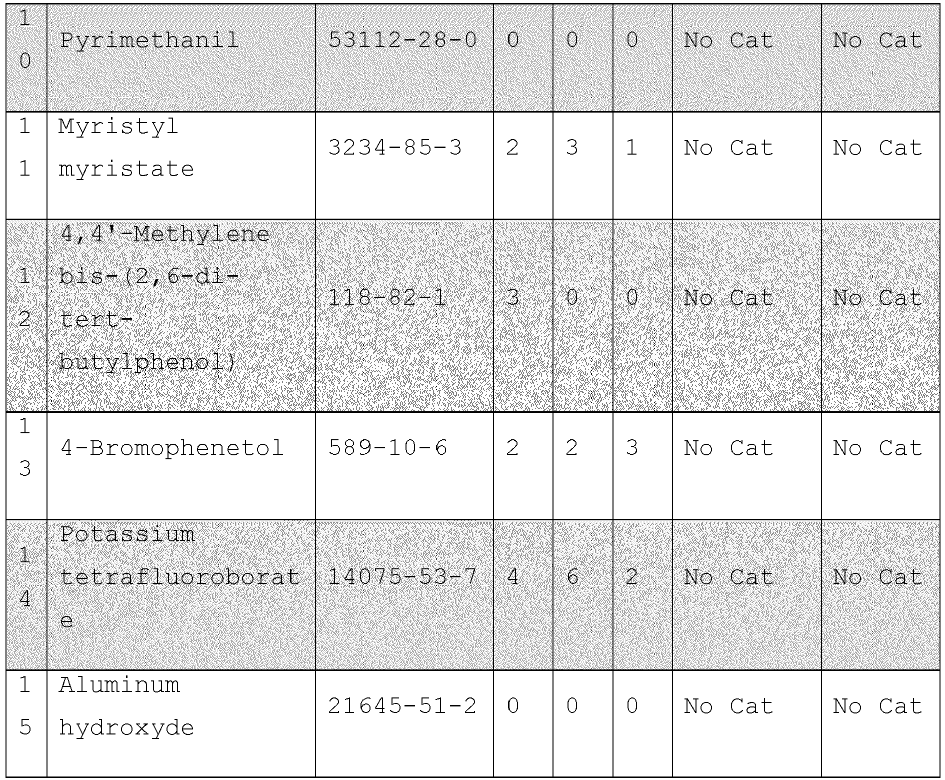

**Table 8: Mesure de la capacité de prédiction du test d'irritation oculaire pour différencier les irritants oculaires (classés comme Cat 1 ou Cat 2) des non irritants (no Cat) sur un set de 15 produits chimiques solides**

| DRAIZE | Classés | No Categorie |
|---|---|---|
| Classés (n) | 7 | 0 |
| No Categorie (n) | 0 | 8 |

| Total (n) | 7 | 8 |
|---|---|---|
| Sensitivité (%) | 100 | |
| Specificité (%) | 100 | |
| Précision (%) | 100 | |

**Table 9 : Analyse par matrice de confusion de la capacité de prédiction du test d'irritation oculaire à séparer entre 3 classes d'irritation (Cat 1, Cat 2 and no Cat) selon les recommandations UN-GHS des 15 produits solides.**

| | | Test d'irritation oculaire | | | | |
|---|---|---|---|---|---|---|
| | | Cat 1 | Cat 2 | No Cat | Classification | « Producer accuracy » (Précision) |
| UNGHS | Cat 1 | 5 | | | 5 | 100% |
| | Cat 2 | | 2 | | 2 | 100% |
| | No Cat | | | 8 | 8 | 100% |
| | « Truth Overall » | 5 | 2 | 8 | 15 | |
| | « User accuracy » (Recall) | 100% | 100% | 100% | | |
| « Overall Accuracy » | 100% | | | | | |
| Kappa | 1 | | | | | |

## Revendications

1. Méthode d'évaluation du potentiel d'irritation oculaire d'un composé test, comprenant les étapes de:
a) mise en contact d'un composé test avec un échantillon cornéal reconstruit in vitro;
b) mesure de l'expression d'au moins deux gènes choisis dans le groupe constitué par : HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.
**caractérisée en ce que** :
- la mise en contact du composé test à l'étape a) est réalisée avec le composé test sous la forme solide et l'étape b) comprend la mesure de l'expression d'au moins deux gènes choisis dans le groupe constitué par : IL-24, IL-23R, DDIT3, MMP8, DLK1, HAS1, CYR61, IL-1R2, CLEC4D, ICAM2, CASP1, MUC13 et MUC4, ou
- la mise en contact du composé test à l'étape a) est réalisée avec le composé test sous la forme liquide et l'étape b) comprend la mesure de l'expression d'au moins deux gènes choisis dans le groupe constitué par : HSP90AA1, CASP1, DLK1, CLEC4D, IL-24, SLIT2, HSPA1A, FSHR, IL-1R2 et CCND1.

2. Méthode selon la revendication 1, **caractérisée en ce qu'**elle comprend une étape supplémentaire c) de détermination d'un index d'irritation oculaire du composé test.

3. Méthode selon la revendication 2, **caractérisée en ce qu'**elle comprend une étape d) de catégorisation dudit composé comme présentant un potentiel d'irritation oculaire selon la valeur de l'index d'irritation oculaire obtenu.

4. Méthode selon l'une des revendications précédentes, **caractérisée** en que l'échantillon cornéal reconstruit in vitro est un échantillon comprenant des cellules épithéliales de cornée immortalisées, cultivées en milieu de culture défini et disposées en couche fine sur une membrane synthétique à l'interface eau-air.

5. Méthode selon l'une des revendications 2 à 4, **caractérisée en ce que** la détermination de l'index d'irritation oculaire du composé comprend l'attribution d'une valeur seuil de surexpression à chaque gène dont l'expression est mesurée.

6. Méthode selon la revendication 5, **caractérisée en ce que** la valeur seuil indiquant une surexpression significative du gène dont l'expression est mesurée peut être comprise en 1,1 et 10.

7. Méthode selon l'une des revendications 5 ou 6 **caractérisée en ce que** la détermination de l'index d'irritation oculaire comprend l'attribution d'une valeur de poids à chaque gène si la valeur seuil de surexpression dudit gène est atteinte.

8. Méthode selon la revendication 7 **caractérisée en ce que** la valeur de poids est choisie dans le groupe de valeurs discrètes échelonnées entre 1 et 10, à savoir choisies dans le groupe consistant en les valeurs 1, 2, 3, 4, 5, 6, 7, 8, 9 et 10.

9. Méthode selon la revendication 7 ou 8 **caractérisée en ce que** l'index d'irritation oculaire est déterminé par l'addition des valeurs de poids des gènes dont l'expression dépasse la valeur seuil de surexpression.

10. Méthode selon l'une des revendications 3 à 9, **caractérisée en ce que** l'étape de catégorisation comprend l'attribution d'une catégorie d'irritation au composé test en fonction de la valeur de l'index d'irritation oculaire obtenu.

## Patentansprüche

1. Verfahren zur Evaluierung des Augenreizpotentials einer Testverbindung, umfassend die Schritte:
a) Inkontaktbringen einer Testverbindung mit einer in vitro rekonstruierten Hornhautprobe;
b) Messen der Expression von mindestens zwei Genen, die ausgewählt sind aus der Gruppe bestehend aus: HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61,
**dadurch gekennzeichnet, dass**:
- das Inkontaktbringen der Testverbindung im Schritt a) mit der Testverbindung in fester Form durchgeführt wird und der Schritt b) das Messen der Expression von mindestens zwei Genen umfasst, die ausgewählt sind aus der Gruppe bestehend aus: IL-24, IL-23R, DDIT3, MMP8, DLK1, HAS1, CYR61, IL-1R2, CLEC4D, ICAM2, CASP1, MUC13 und MUC4, oder
- das Inkontaktbringen der Testverbindung im Schritt a) mit der Testverbindung in flüssiger Form durchgeführt wird und der Schritt b) das Messen der Expression von mindestens zwei Genen umfasst, die ausgewählt sind aus der Gruppe bestehend aus: HSP90AA1, CASP1, DLK1, CLEC4D, IL-24, SLIT2, HSPA1A, FSHR, IL-1R2 und CCND1.

2. Verfahren nach Anspruch 1, **dadurch gekennzeichnet, dass** es einen zusätzlichen Schritt c) eines Bestimmens eines Augenreizindexes der Testverbindung umfasst.

3. Verfahren nach Anspruch 2, **dadurch gekennzeichnet, dass** es einen Schritt d) eines Kategorisierens der Verbindung als ein Augenreizpotential aufweisend gemäß dem erhaltenen Augenreizindex umfasst.

4. Verfahren nach einem der vorhergehenden Ansprüche, **dadurch gekennzeichnet, dass** die in vitro rekonstruierte Hornhautprobe eine Probe ist, die immortalisierte Hornhautepithelzellen umfasst, die in einem definierten Kulturmedium kultiviert und in einer dünnen Schicht auf einer synthetischen Membran an der Wasser-Luft-Grenzfläche angeordnet wurden.

5. Verfahren nach einem der Ansprüche 2 bis 4, **dadurch gekennzeichnet, dass** das Bestimmen des Augenreizindexes der Verbindung das Zuweisen eines Überexpressionsschwellenwerts zu jedem Gen, dessen Expression gemessen wird, umfasst.

6. Verfahren nach Anspruch 5, **dadurch gekennzeichnet, dass** der Schwellenwert, der eine signifikante Überexpression des Gens angibt, dessen Expression gemessen wird, zwischen 1,1 und 10 liegen kann.

7. Verfahren nach einem der Ansprüche 5 oder 6, **dadurch gekennzeichnet, dass** das Bestimmen des Augenreizindexes das Zuweisen eines Gewichtswerts zu jedem Gen umfasst, wenn der Überexpressionsschwellenwert des Gens erreicht wird.

8. Verfahren nach Anspruch 7, **dadurch gekennzeichnet, dass** der Gewichtswert aus der Gruppe von diskreten abgestuften Werten zwischen 1 und 10 ausgewählt ist, die nämlich aus der Gruppe bestehend aus den Werten 1, 2, 3, 4, 5, 6, 7, 8, 9 und 10 ausgewählt sind.

9. Verfahren nach Anspruch 7 oder 8, **dadurch gekennzeichnet, dass** der Augenreizindex durch die Addition von Gewichtswerten von Genen, dessen Expression den Überexpressionsschwellenwert übersteigt, bestimmt wird.

10. Verfahren nach einem der Ansprüche 3 bis 9, **dadurch gekennzeichnet, dass** der Schritt des Kategorisierens das Zuweisen einer Reizkategorie zu der Testverbindung in Abhängigkeit von dem erhaltenen Wert des Augenreizindexes umfasst.

## Claims

1. A method for evaluating the eye irritation potential of a test compound, comprising the steps of:
a) bringing a test compound into contact with an in vitro reconstructed corneal sample;
b) measuring the expression of at least two genes selected from the group consisting of: HSP90AA1, COL7A1, NOS3, MMP8, CASP1, ELN, IL-23R, DLK1, CLEC4D, IL-24, CCL22, SLIT2, ICAM2, MUC13, HSPA1A, FSHR, IL-1R2, ALB, CCND1, CXCL1, CXCR1, KL, COL6A2, MUC4, DDIT3, MMP3, HAS1, IRF1, CYR61.
**characterized in that**:
- the bringing into contact of the test compound in step a) is carried out with the test compound in solid form and step b) comprises measuring the expression of at least two genes selected from the group consisting of: IL-24, IL-23R, DDIT3, MMP8, DLK1, HAS1, CYR61, IL-1R2, CLEC4D, ICAM2, CASP1, MUC13 and MUC4, or
- the bringing into contact of the test compound in step a) is carried out with the test compound in liquid form and step b) comprises measuring the expression of at least two genes selected from the group consisting of: HSP90AA1, CASP1, DLK1, CLEC4D, IL-24, SLIT2, HSPA1A, FSHR, IL-1R2 and CCND1.

2. The method as claimed in claim 1, **characterized in that** it comprises an additional step c) of determining an eye irritation index of the test compound.

3. The method as claimed in claim 2, **characterized in that** it comprises a step d) of categorizing said compound as having an eye irritation potential according to the value of the eye irritation index obtained.

4. The method as claimed in one of the preceding claims, **characterized in that** the in vitro reconstructed corneal sample is a sample comprising immortalized corneal epithelial cells, grown in defined culture medium and arranged in a thin layer on a synthetic membrane at the water-air interface.

5. The method as claimed in one of claims 2 to 4, **characterized in that** the determination of the eye irritation index of the compound comprises assigning an overexpression threshold value to each gene whose expression is measured.

6. The method as claimed in claim 5, **characterized in that** the threshold value indicating significant overexpression of the gene whose expression is measured may be comprised between 1.1 and 10.

7. The method as claimed in one of claims 5 or 6, **characterized in that** the determination of the eye irritation index comprises assigning a weight value to each gene if the overexpression threshold value of said gene is reached.

8. The method as claimed in claim 7, **characterized in that** the weight value is selected from the group of discrete values ranging from 1 and 10, namely selected from the group consisting of the values 1, 2, 3, 4, 5, 6, 7, 8, 9 and 10.

9. The method as claimed in claim 7 or 8, **characterized in that** the eye irritation index is determined by adding the weight values of genes whose expression exceeds the overexpression threshold value.

10. The method as claimed in one of claims 3 to 9, **characterized in that** the categorization step comprises assigning an irritation category to the test compound based on the value of the eye irritation index obtained.
